# EUROPEAN PATENT APPLICATION

(11) **EP 4 847 495 A1**
(43) Date of publication of application: **02.12.2026**
(21) Application number: 25744745.8
(22) Date of filing: 24.01.2025
(51) Int. Cl.: C07D 333/50, C07D 307/77, C07F 5/00, C07K 1/00, G01N 33/68, A61K 45/00, A61K 31/343, A61P 25/28

(54) **BENZOHETEROCYCLIC COMPOUND AND USE THEREOF**

(30) Priority: 24.01.2024 CN 202410102966; 12.03.2024 CN 202410281955
(71) Applicant: ShanghaiTech University, Shanghai 201210 (CN)
(72) Inventor: ZHAO, Jian, Shanghai 201210 (CN); LUO, Zonghua, Shanghai 201210 (CN); GUO, Na, Shanghai 201210 (CN); PEI, Gang, Shanghai 201210 (CN); PU, Suyun, Shanghai 201210 (CN)
(74) Representative: Synergy IP Group AG
(86) International application number: PCT/CN2025/074897
(87) International publication number: WO 2025/157282

(57) **Abstract**

Provided in the present invention are a benzoheterocyclic compound and the use thereof. Specifically, provided in the present invention is a compound represented by formula D, a pharmaceutically acceptable salt thereof, a solvate thereof or a solvate of the pharmaceutically acceptable salt thereof, wherein X₂ is O, S or Se and Z₂ is F-18 or I-124. The benzoheterocyclic compound of the present invention can inhibit the formation of Aβ polymer, promote the depolymerization of Aβ polymer, inhibit abnormal activation of a microglial cell, and improve memory, thereby realizing the effects of improving cognitive impairment and treating AD. In addition, the benzoheterocyclic compound provided in the present invention is used as Aβ ligands. These ligands have high affinity and can specifically bind to Aβ protein in the brain. Further, these ligands can be labeled with radioisotopes F-18 and Ga-68 and can be used to observe and measure the distribution and accumulation of Aβ protein in the brain by means of the non-invasive PET imaging method.

## Description

The present application claims priority to Chinese Patent Application No. 202410102966X, filed on January 24, 2024, and Chinese Patent Application No. 2024102819552, filed on March 12, 2024. The full contents of the aforesaid Chinese patent applications are incorporated herein by reference in their entirety.

### TECHNICAL FIELD

The present application relates to a benzoheterocyclic compound and use thereof.

### BACKGROUND

Alzheimer's disease (AD), also referred to as Alzheimer disease, is a chronic progressive neurodegenerative disorder of the nervous system and one of the most common causes of senile dementia. This disease primarily affects brain function, leading to a gradual loss of intellectual functions such as cognition and memory. The major pathological features of AD are the deposition of two abnormal proteins in brain tissue: β-amyloid protein (Aβ) and Tau protein. The Aβ protein aggregates in the brain to form amyloid plaques, while the Tau protein accumulates within neurons to form neurofibrillary tangles. The accumulation of these abnormal proteins leads to a progressive loss of neuronal function and connectivity, ultimately resulting in brain atrophy. At present, there is no curative treatment for Alzheimer's disease.

The aggregation of amyloid β-protein (Aβ) is one of the most important hypotheses in the pathogenesis of AD, and inhibition of Aβ aggregation has been proven to be an effective therapeutic strategy for AD. Existing studies have demonstrated that soluble Aβ oligomers are the major source of neurotoxicity and play a critical role in the onset and progression of AD by affecting neuronal membrane ion channels, inducing oxidative stress, and activating glial cells to trigger inflammatory responses. Aβ exists in multiple forms in the human body, predominantly Aβ42 and Aβ40. Among them, Aβ42 has become a research focus due to its higher neurotoxicity and greater propensity to aggregate. Other Aβ species, such as N-terminally truncated and modified forms including N3pE variants, are also considered to be highly associated with AD pathology. Therefore, clearance of Aβ has become one of the most widely investigated therapeutic mechanisms for AD. Aβ monoclonal antibodies are the most extensively validated class of novel therapeutic agents for AD and are currently the only antibody-based therapies approved for marketing. Multiple clinical studies have shown that Aβ monoclonal antibodies can reduce Aβ plaque burden and, to some extent, slow the decline in cognitive and functional abilities. Although the clinical development of certain monoclonal antibodies has been discontinued due to limited blood-brain barrier penetration, insufficient therapeutic efficacy, or adverse events such as cerebral edema or microhemorrhage, more than ten Aβ monoclonal antibodies are still undergoing clinical trials at different stages.

Almost all drug development approaches targeting Aβ adopt Aβ prepared under specific conditions as drug targets. To screen and develop drug molecules capable of specifically binding to various forms of Aβ, diverse Aβ oligomers with different sequences, different degrees of aggregation, and aggregates mixed in different ratios are required as screening substrates. Overall, preparation methods of Aβ can be divided into three categories. The first category employs in vivo-derived Aβ obtained by extraction and purification. This method can maximally obtain various Aβ aggregates close to the real in vivo state. However, extraction from the brain tissue of deceased AD patients is obviously impractical and cannot be applied to drug screening. Aβ derived from transgenic mice is limited by genotype constraints, making it difficult to reflect the complex Aβ isoforms in the human brain. Meanwhile, its extraction and purification procedures are cumbersome and labor-intensive, and the extraction quality is susceptible to multiple conditions, rendering it unsuitable for large-scale drug screening. The second category involves extraction and purification of Aβ expressed via transfected cells. This method enables easier acquisition of sufficient Aβ in the required quantity compared with the first method. However, it requires strict aseptic biological culture and operation, as well as a relatively complex process such as plasmid construction. Meanwhile, it cannot conveniently and rapidly obtain multiple Aβ sequences and mixed forms thereof simultaneously. The third category adopts artificially synthesized Aβ. The technology of solid-phase peptide synthesis is highly mature. This technology can be used to obtain various Aβ sequences rapidly, conveniently and with high purity. However, there is still no unified standard for how to use these Aβ sequences to prepare various Aβ oligomers for drug screening. The inventors have found that numerous methods for preparing Aβ aggregates have been reported in various literature. Most of these methods are merely based on simple buffer solution or culture medium conditions, lacking systematic comparison and analysis of the morphology and aggregation state of Aβ oligomers prepared under different conditions. Unlike other common protein targets with relatively defined binding sites and three-dimensional structures, Aβ does not have fixed structural characteristics. The polymorphism of Aβ greatly increases the difficulty of targeted research thereon. Since multiple Aβ variants with different lengths and terminal modifications exist in vivo, the aggregation properties of respective Aβ reported in the prior art differ significantly. Different incubation conditions also exert a remarkable influence on the aggregation behavior of Aβ. Moreover, the aggregation process of Aβ itself exhibits dynamic aggregation with varying degrees, morphologies and sizes, showing extremely high heterogeneity. Accordingly, the success rate of anti-Aβ drug research and development is extremely low.

Some drugs and therapeutic measures can help alleviate symptoms and improve quality of life of patients. Therefore, early diagnosis and intervention are critical to the management of Alzheimer's disease.

To date, the diagnosis of Alzheimer's disease still mainly relies on clinical standard evaluation, in vivo brain tissue biopsy, and post-mortem tissue research. However, researchers have explored multiple fields for the development of in vivo diagnostic methods for Alzheimer's disease, including genetic testing, immunoassay approaches, and imaging techniques. In the field of genetic testing, researchers are committed to identifying genetic factors associated with Alzheimer's disease. Mutations of specific genes are associated with the risk of developing AD. Therefore, genetic testing can be used to evaluate the underlying disease risk of individuals. Such genetic testing provides a potential approach for early diagnosis and facilitates early intervention and treatment. On the other hand, immunoassay methods are widely applied to the detection of AD biomarkers in body fluids. By detecting changes in specific proteins such as the Aβ protein and the Tau protein in cerebrospinal fluid or blood, the diagnosis and disease progression of AD can be evaluated. These biomarkers hold potential value in the diagnosis and condition monitoring of AD. In addition, the imaging technology has been also extensively studied for non-invasive diagnosis of AD. Positron Emission Tomography (PET) is a commonly used imaging technology, which can employ specific tracers to label the Aβ protein and the Tau protein in the brain. These imaging methods enable the visualization of pathological changes associated with AD and facilitate early diagnosis and disease monitoring.

At present, AD is clinically diagnosed routinely using various approaches, including plasma biomarker testing, cerebrospinal fluid biochemical metabolic examination, cognitive function assessment, and medical imaging. Blood tests and brain imaging can exclude other potential causes of dementia symptoms, such as cardiovascular and cerebrovascular diseases, tumors, and deficiencies of certain vitamins. Cognitive function assessments are generally associated with Alzheimer's disease but cannot directly confirm the diagnosis of Alzheimer's disease. A definitive diagnosis of Alzheimer's disease relies on pathological examination of brain tissue. The level of Aβ in cerebrospinal fluid is evaluated by measuring the content of Aβ42 or the ratio of Aβ40 to Aβ42. However, cerebrospinal fluid collection requires lumbar puncture, which is poorly tolerated by patients and not suitable for routine clinical diagnosis and screening of AD. Compared with the aforementioned methods, Aβ-PET imaging is performed by using specific Aβ radioactive tracers. PET imaging can better evaluate the extent and location of Aβ deposition, and exhibits higher sensitivity and specificity for visualizing Aβ. More importantly, PET imaging features a more standardized procedure and exhibits high intra-individual reproducibility, which is essential for comparing data across studies and longitudinal changes.

Fluorine-18-labeled fluorodeoxyglucose (¹⁸F-FDG) is one of the earliest PET tracers used for the diagnosis of AD. However, the uptake of ¹⁸F-FDG by tissues is highly dependent on the metabolic activity of tissues. Glucose is also taken up by many tissues under normal physiological conditions, particularly the brain, heart, and skeletal muscle. Accordingly, relatively high background signals may be generated in these regions, which fail to accurately reflect the pathological changes associated with AD. Therefore, this tracer is no longer used as an imaging agent for AD diagnosis. In 2002, in vivo Aβ-PET imaging was first performed on AD patients. A C-11 labeled Pittsburgh compound B (¹¹C-PIB) is an Aβ-PET molecular imaging tracer designed based on the Aβ stain Thioflavin T (ThT). Imaging results at nanomolar injection concentrations have verified that ¹¹C-PIB can specifically bind to Aβ and reflect the location and concentration of Aβ deposition in living brain tissue. It is shown that the radioactivity in the frontal lobe, temporal lobe and parietal lobe of AD patients is significantly increased, which is consistent with the Aβ plaque deposition areas identified in autopsy pathology. However, the short half-life of the carbon-11 isotope (T₁/₂ = 20 min) limits the application of this tracer to only PET research centers equipped with a cyclotron. On this basis, molecular imaging tracers labeled with F-18 (¹⁸F) featuring a longer half-life (T₁/₂ = 110 min) have been developed for broader applications. At present, the three Aβ-PET molecular imaging tracers approved by the FDA for the early clinical diagnosis of AD are all F-18 radiolabeled molecular probes, namely ¹⁸F-Florbetapir, ¹⁸F-Florbetaben and ¹⁸F-Flutemetamol. However, the disadvantages of such PET molecular imaging tracers lie in poor stability, high uptake in cerebral white matter, and unsatisfactory sensitivity and specificity for diagnosis that need to be further improved. As an ideal Aβ-PET molecular imaging tracer, it is to have the following characteristics: (1) appropriate lipophilicity to enable crossing of the blood-brain barrier, entry into the brain, and binding to Aβ; (2) high specific binding affinity to Aβ with low non-specific binding; (3) good in vivo stability to reduce non-specific signals from metabolites in the brain; and (4) simple and efficient preparation to meet the requirements of clinical application.

Therefore, the development of novel Aβ-targeted drugs and PET molecular imaging tracers is of great significance for the specific diagnosis and treatment of AD.

### SUMMARY

To solve the technical problem in the prior art that existing Aβ-PET molecular imaging tracers are single in structure, and AD therapeutic drugs are few in type and poor in effect, the present invention is to provide a benzoheterocyclic compound and use thereof. The benzoheterocyclic compound of the present invention can inhibit the formation of Aβ aggregates, promote the disaggregation of Aβ aggregates, inhibit the abnormal activation of microglia, and improve memory, thereby ameliorating cognitive impairment and achieving the therapeutic effect on Alzheimer's disease. Meanwhile, the benzoheterocyclic compound provided by the present application serves as Aβ ligands. Such ligands possess high affinity and can specifically bind to Aβ protein in the brain. Further, these ligands can be labeled with radioactive isotopes F-18 and Ga-68. Through a non-invasive PET imaging approach, the distribution and accumulation of Aβ protein in the brain can be observed and quantified.

The present invention provides a compound represented by formula A, a pharmaceutically acceptable salt thereof, a solvate thereof, or a solvate of the pharmaceutically acceptable salt thereof: 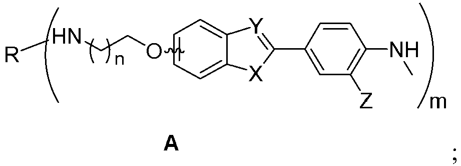
where R is composed of a chelating group and a radionuclide;
the chelating group is unmodified or modified with a cell-penetrating peptide;
the radionuclide is a diagnostic radionuclide;
m is 1 or 2;
each X is independently O, S, or Se;
each Y is independently CH or N;
each Z is independently H, F, F-18, Cl, Br, or I;
each n is independently 1, 2, 3, or 4.

In some implementations, in the compound represented by formula A, the pharmaceutically acceptable salt thereof, the solvate thereof, or the solvate of the pharmaceutically acceptable salt thereof, some groups can be as defined below, and other groups can be as defined in any one implementation of the present invention (hereinafter referred to as some implementations). X is O or S.

In some implementations, Z is F or H.

In some implementations, n is 3 or 4.

In some implementations, the cell-penetrating peptide is HAIYPRH (SEQ ID NO: 1), THRPPMWSPVWP (SEQ ID NO: 2), TFFYGGSRGKRNNFKTEEY (SEQ ID NO: 3), AGILKRW (SEQ ID NO: 4), CGNKRTR (SEQ ID NO: 5), LRKLRKRLLR (SEQ ID NO: 6) or YGRKKRRQRRR (SEQ ID NO: 7).

In some implementations, the chelating group is

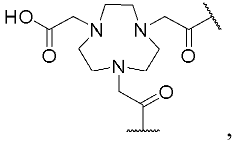

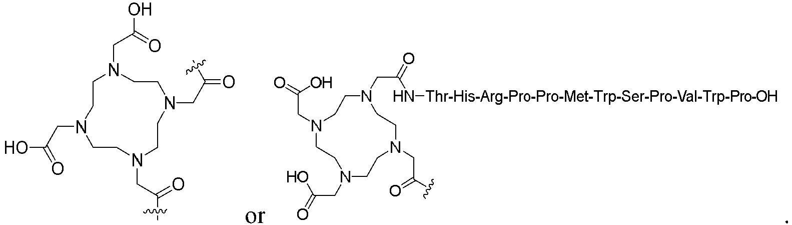

In some implementations, the radionuclide is selected from Cu-64 and Ga-68, for example Ga-68.

In some implementations, the compound represented by formula A is represented by formula A-1:

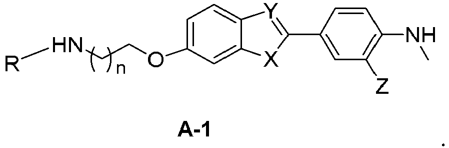

In some implementations, the compound represented by formula A is selected from any one of the following compounds:

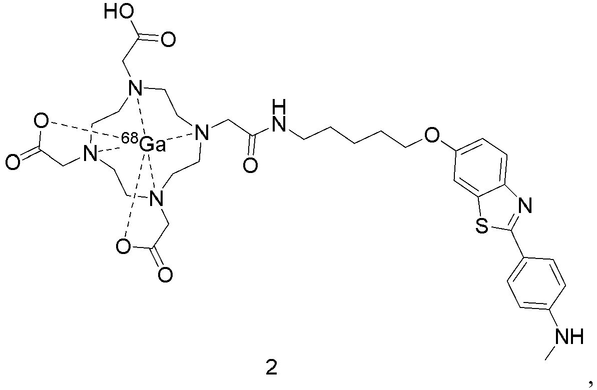

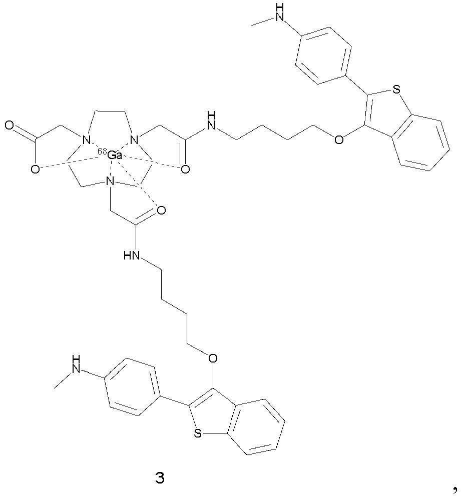

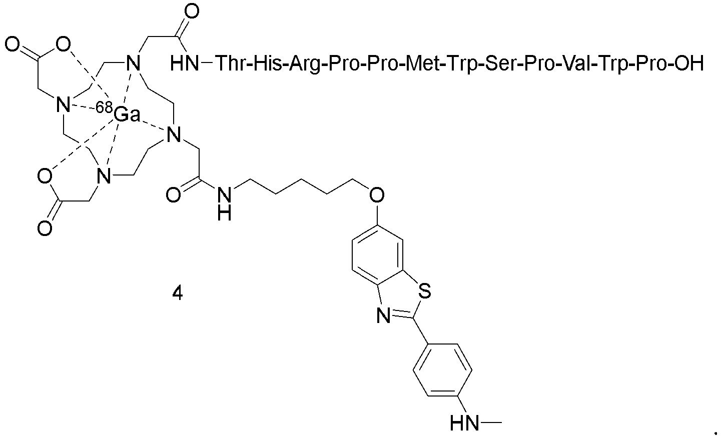

The present invention further provides a compound represented by formula B, a pharmaceutically acceptable salt thereof, a solvate thereof, or a solvate of the pharmaceutically acceptable salt thereof: 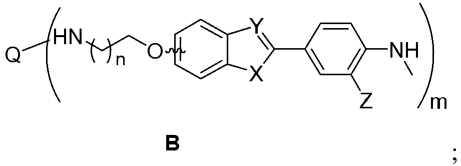 where X, Y, Z, m, and n are as defined in any one of implementations, and Q is a chelating group as defined in any one of implementations.

In some implementations, the compound represented by formula B is any one of the following compounds:

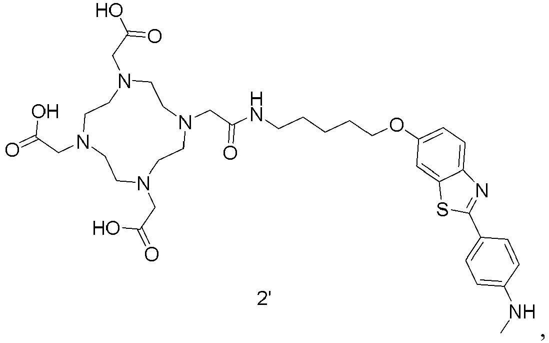

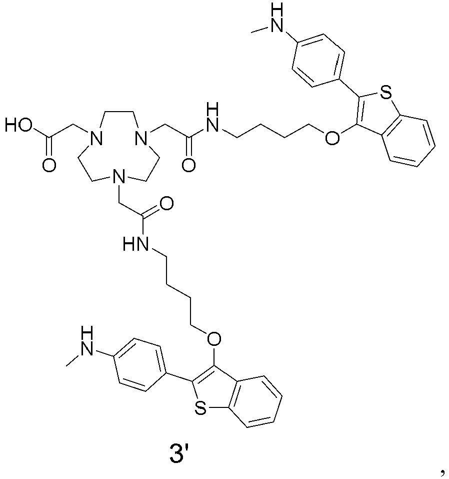

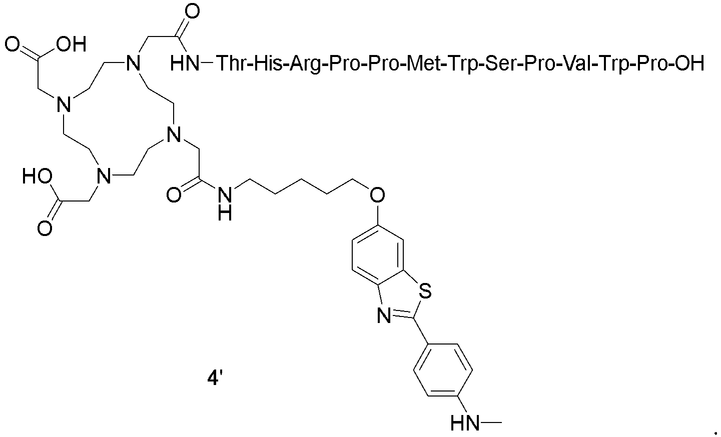

The present invention further provides a compound represented by formula C, a pharmaceutically acceptable salt thereof, a solvate thereof, or a solvate of the pharmaceutically acceptable salt thereof: 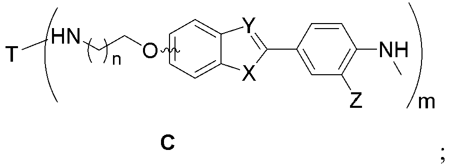
where X, Y, Z, m, and n are as defined in any one of implementations;
T is composed of a chelating group and a non-radionuclide, and the chelating group is as defined in any one of implementations.

In some implementations, the non-radionuclide is Cu or Ga, for example Ga.

In some implementations, the compound represented by formula C is

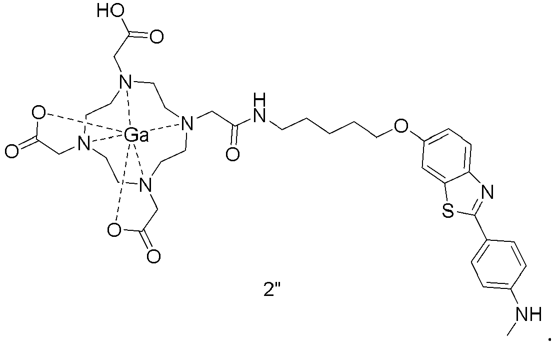

The present invention further provides a compound represented by formula D, a pharmaceutically acceptable salt thereof, a solvate thereof, or a solvate of the pharmaceutically acceptable salt thereof: 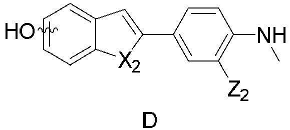
where X₂ is O, S, or Se;
Z₂ is F-18 or I-124.

In some implementations, X₂ is O or S.

In some implementations, Z₂ is F-18.

In some implementations, the compound represented by formula D is

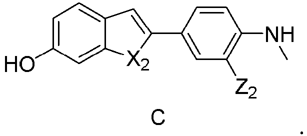

In some implementations, the compound represented by formula D is

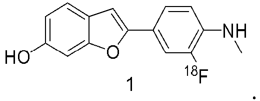

The present invention further provides a compound represented by formula E, a pharmaceutically acceptable salt thereof, a solvate thereof, or a solvate of the pharmaceutically acceptable salt thereof: 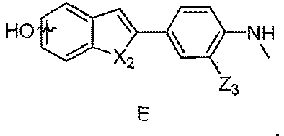
where X₂ is as defined in any one of implementations;
Z₃ is F or I.

In some implementations, the Z₃ is F.

In some implementations, the compound represented by formula E is

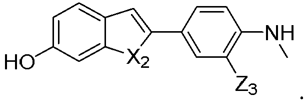

In some implementations, the compound represented by formula E is

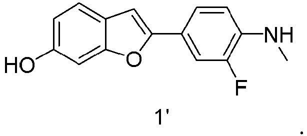

The present invention further provides a pharmaceutical composition, including the compound, the pharmaceutically acceptable salt thereof, the solvate thereof, or the solvate of the pharmaceutically acceptable salt thereof according to any one of aforementioned implementations, and a pharmaceutically acceptable excipient.

The present invention further provides a kit, including the compound, the pharmaceutically acceptable salt thereof, the solvate thereof, or the solvate of the pharmaceutically acceptable salt thereof according to any one of aforementioned implementations, or the above pharmaceutical composition and instructions for use.

The present invention further provides use of the compound represented by formula A or the compound represented by formula D in preparation of a PET molecular probe.

In some implementations, the PET molecular probe is used for diagnosis of Alzheimer's disease.

In some implementations, the PET molecular probe is used for imaging Aβ protein-binding.

The present invention further provides use of the compound, the pharmaceutically acceptable salt thereof, the solvate thereof, or the solvate of the pharmaceutically acceptable salt thereof described above in preparation of a medicament for preventing and/or treating Alzheimer's disease. For example, use of the compound represented by formula E, the pharmaceutically acceptable salt thereof, the solvate thereof, or the solvate of the pharmaceutically acceptable salt thereof in preparation of a medicament for preventing and/or treating Alzheimer's disease.

The present invention further provides use of the compound, the pharmaceutically acceptable salt thereof, the solvate thereof, or the solvate of the pharmaceutically acceptable salt thereof described above in preparation of an anti-Aβ drug, where the substance V is as defined in claim 15; and preferably, the anti-Aβ drug exerts a therapeutic effect by disaggregating Aβ oligomers or by preventing the formation of Aβ oligomers.

The present invention further provides the compound, the pharmaceutically acceptable salt thereof, the solvate thereof, or the solvate of the pharmaceutically acceptable salt thereof described above, which perform one or more of the following functions: (i) inhibiting formation of Aβ oligomers; (ii) promoting disaggregation of Aβ oligomers; and (iii) binding to Aβ oligomers.

Another objective of the present invention is to provide a method for preparing an Aβ aggregate.

Another objective of the present invention is to provide a target for screening anti-Aβ drug.

Another objective of the present invention is to provide a method for screening an anti-Aβ drug.

To solve the above technical problems, in a first aspect of the present invention, provided is a method for preparing an Aβ aggregate, including the following steps:
S1, dissolving an Aβ lyophilized powder and drying to remove a solvent to obtain an Aβ peptide film;
S2, mixing the Aβ peptide film with a buffer solution to obtain an Aβ solution with a concentration of 0.2-100 µM;
S3, incubating the Aβ solution to obtain an Aβ aggregate; and
where in step S1, the Aβ lyophilized powder is Aβ40, Aβ42, N3pE42, Aβ38, or a combination thereof;
in step S2, the buffer solution is selected from at least one of calcium- and magnesium-free Dulbecco's phosphate-buffered saline (DPBS), an HBS buffer solution containing 8-12 mM HEPES and 140-160 mM NaCl, artificial cerebrospinal fluid (aCSF), DMEM, FBS, endosome-mimicking fluid, and lysosome-mimicking fluid;
in step S3, the incubation temperature is 30 to 43°C, the incubation shaking speed is 0 to 2500 rpm, and the incubation time is not less than one day.

In some preferred implementations, in step S1, the solvent is hexafluoroisopropanol.

In some preferred implementations, in step S3, the incubation time is 1 to 7 days.

In some preferred implementations, in step S3, the incubation is performed in a constant-temperature homogenizer, and a rotational speed of the constant-temperature homogenizer is 0 to 2500 rpm.

In some preferred implementations, a concentration of the Aβ solution is 0.5-50 µM, more preferably 1-20 µM.

In some preferred implementations, the buffer solution is calcium- and magnesium-free Dulbecco's phosphate-buffered saline (DPBS), the HBS buffer solution containing 8-12 mM HEPES and 140-160 mM NaCl, or the artificial cerebrospinal fluid (aCSF).

In some preferred implementations, the endosome-mimicking fluid is a mixed solution of a sodium citrate buffer solution with a concentration of 18-22 mM and pH of 5.5, a NaCl solution with a concentration of 75-85 mM, and a KCl solution with a concentration of 18-22 mM.

In some preferred implementations, the lysosome-mimicking fluid is a mixed solution of a sodium citrate buffer solution with a concentration of 18-22 mM and pH of 4.5, a NaCl solution with a concentration of 45-55 mM, and a KCl solution with a concentration of 45-55 mM.

In some preferred implementations, the Aβ lyophilized powder is a combination of Aβ40 and Aβ42, or a combination of Aβ40 and N3pE42, or a combination of Aβ40, Aβ42, and N3pE42. Preferably, in the combination, a mole percentage of Aβ40 is not less than 50%. In some preferred implementations, in the combination, a molar ratio of Aβ40 and other Aβ is (5-9): (1-5).

In some preferred implementations, the Aβ lyophilized powder is a combination of Aβ40 and Aβ42, where a molar ratio of Aβ40 to Aβ42 is 9: 1, 8: 2, 7: 3, 6: 4 or 5: 5.

In some preferred implementations, the Aβ lyophilized powder is a combination of Aβ40 and N3pE42, where a molar ratio of Aβ40 to N3pE42 is 9: 1. In some preferred implementations, the Aβ lyophilized powder is a combination of Aβ40, Aβ42, and N3pE42, where a molar ratio of Aβ40, Aβ42, and N3pE42 is 5: 4: 1.

In some preferred implementations, the incubation temperature is 36-41°C. The incubation shaking speed is 1500 to 2500 rpm.

In a second aspect of the present invention, provided is use of an Aβ aggregate as a target for screening an anti-Aβ drug, where the Aβ aggregate is prepared by the method in the first aspect of the present invention.

In a third aspect, provided is a target for screening an anti-Aβ drug, where the target is an Aβ aggregate, and the Aβ aggregate is prepared by the method in the first aspect of the present invention.

In a fourth aspect, provided is a method for screening an anti-Aβ drug, including the following step: screening the anti-Aβ drug using the target in the third aspect of the present invention.

In some preferred implementations, the method includes the following step: screening a drug capable of disaggregating Aβ aggregates or inhibiting Aβ aggregation to form aggregates, where the drug is selected from at least one of an antibody, a polypeptide, and a small molecule compound.

In a fifth aspect, provided is an anti-Aβ drug, which is screened by the method in the fourth aspect of the present invention.

In some preferred implementations, the compound is a compound represented by formula I,

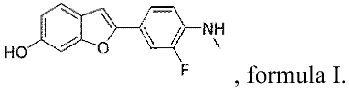

In a sixth aspect, provided is use of a compound represented by formula I for in vitro non-therapeutic use, (i) inhibiting formation of Aβ oligomers; (ii) promoting disaggregation of Aβ oligomers; and/or (iii) binding to Aβ oligomers;

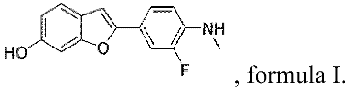

Compared with the prior art, the present invention has the following advantages:
(1) The screening method provided by the present invention adopts different Aβ aggregates prepared by simulating in-vivo environments and conditions as targets, and can screen anti-Aβ candidate drugs more accurately and efficiently;
(2) The Aβ target involved in the screening method provided by the present invention undergoes aggregation and incubation under conditions simulating different in vivo microenvironments, without requiring any labeling or modification thereof. Meanwhile, the screened drug molecules also do not need any modification or labeling;
(3) The screening method provided by the present invention can more intuitively screen out anti-Aβ candidate drugs with good activity;
(4) The screening method provided by the present invention is not limited to a certain type of drugs, and can be widely applied to different drug types such as antibodies, polypeptides and small molecules. It can truly reflect the interaction between the target and such drug molecules, and greatly reduce the probability of occurrence of false positives, false negatives or incorrect binding modes.

The compound provided by the present invention has the following advantageous technical effects: (1) The F-18 radiochemical synthesis in the present invention achieves a high labeling rate with mild labeling conditions, and the target compound is easy to prepare; (2) The Ga-68 radiochemical synthesis of the present invention features high labeling yield, short labeling time and simple operation. A portable Ge-Ga generator can be adopted, and sufficient dose preparation can be realized without relying on a large cyclotron, which facilitates the production and popularization and application in primary hospitals; and (3) The novel PET imaging agent of the present invention possesses favorable in vitro and in vivo biological activities and excellent in vivo pharmacokinetic properties, which can overcome the deficiencies of existing PET imaging agents; (4) The compound provided by the present invention is capable of inhibiting the formation of Aβ oligomers, and is superior to the commercially available drug Donepezil; (5) The compound provided by the present invention promotes disaggregation of Aβ oligomers, and is superior to the commercially available drug Donepezil; (6) The compound provided by the present invention has a good binding activity with the Aβ oligomers; (7) The compound provided by the present invention is high in activity to reduce Aβ plaques; (8) The compound provided by the present application exhibits a significant effect in reducing the activation level of microglia in the brain of AD mice, and is capable of alleviating neuroinflammation; and (9) The compound provided by the present application can improve the learning and memory functions of AD mice, and is superior to the commercially available drug Donepezil.

### Terms

Unless otherwise specified, the definitions of groups and terms recited in the specification and claims of the present invention, including definitions given by way of examples, exemplary definitions, preferred definitions, definitions recorded in tables, and definitions of specific compounds in the examples, may be arbitrarily combined with one another. Any definitions of groups and compound structures resulting from such combinations shall fall within the scope disclosed in the specification of the present invention.

The term "cell-penetrating peptide" refers to a short peptide capable of carrying the compound across the blood-brain barrier, and the membrane-penetrating capacity is independent of classical endocytosis. The cell-penetrating peptide includes, but is not limited to, HAIYPRH (SEQ ID NO: 1), THRPPMWSPVWP (SEQ ID NO: 2), TFFYGGSRGKRNNFKTEEY (SEQ ID NO: 3), AGILKRW (SEQ ID NO: 4), CGNKRTR (SEQ ID NO: 5), LRKLRKRLLR (SEQ ID NO: 6), YGRKKRRQRRR (SEQ ID NO: 7) or other polypeptides.

The term "treatment" means administration of a compound or formulation described herein to prevent, ameliorate or eliminate a disease or one or more symptoms associated therewith, and includes:
(i) preventing the onset of a disease or disease state in mammals, particularly when such mammals are susceptible to the disease state but have not yet been diagnosed as suffering therefrom;
(ii) inhibiting a disease or disease state, i.e., arresting the progression thereof;
(iii) alleviating a disease or disease state, i.e., bringing about the regression thereof.

Those skilled in the art should understand that, after having known the structures of the compounds of the present invention, the compounds of the present invention can be obtained using commercially available raw materials or well-known raw materials by a variety of methods well known in the art, for example, with reference to the methods described in the Examples of the present invention. These methods are all included in the present invention.

### BRIEF DESCRIPTION OF DRAWINGS

FIG. 1 shows an UV absorption peak of a compound 2" (i.e., a [⁶⁹Ga] compound 2) at 365 nm.
FIG. 2 shows a radiation signal peak of a compound 2 (i.e., a [⁶⁸Ga] compound 2).
FIG. 3 shows an autoradiography imaging result of a [⁶⁸Ga] compound 2 on a brain section of an APP/PS1 mouse.
FIG. 4 shows a quantitative analysis result of autoradiography imaging of the [⁶⁸Ga] compound 2.
FIG. 5 shows PET/CT images of the [⁶⁸Ga] compound 2 in brains of AD model mice (APP/PS1) and wild-type (WT) mice.
FIG. 6 shows a quantitative analysis of the [⁶⁸Ga] compound 2 taken up in brains of AD model mice (APP/PS1) and wild-type (WT) mice.
FIG. 7 is a representative atomic force microscope (AFM) image of typical Aβ oligomers according to an embodiment of the present invention (left: Aβ42 oligomers; right: Aβ42 plaques).
FIG. 8 is a representative AFM image according to an embodiment of the present invention, left: Aβ40; right: Aβ42 (50 µM, 37°C, 2000 rpm, incubated in HBS for 24 h).
FIG. 9 is a representative AFM image according to an embodiment of the present invention, left: Aβ40; right: Aβ42 (10 µM, 37°C, 2000 rpm, incubated in HBS for 24 h).
FIG. 10 is a representative AFM according to an embodiment of the present invention, left: Aβ40; right: Aβ42 (2 µM, 37°C, 2000 rpm, incubated in HBS for 24 h).
FIG. 11 is a representative AFM image according to an embodiment of the present invention, left: Aβ40; right: Aβ42 (50 µM, 37°C, 2000 rpm, incubated in HBS for 1 week).
FIG. 12 is a representative AFM image according to an embodiment of the present invention, left: Aβ40; right: Aβ42 (50 µM, 37°C, 2000 rpm, incubated in aCSF for 24 h).
FIG. 13 is a representative AFM image according to an embodiment of the present invention, left: Aβ40; right: Aβ42 (10 µM, 37°C, 2000 rpm, incubated in aCSF for 24 h).
FIG. 14 is a representative AFM image according to an embodiment of the present invention, left: Aβ40; right: Aβ42 (2 µM, 37°C, 2000 rpm, incubated in aCSF for 24 h).
FIG. 15 is a representative AFM image according to an embodiment of the present invention, left: Aβ40; right: Aβ42 (50 µM, 37°C, 2000 rpm, incubated in aCSF for 1 week).
FIG. 16 is a representative AFM image according to an embodiment of the present invention, left: Aβ40; right: Aβ42 (10 µM, 37°C, 2000 rpm, incubated in aCSF for 1 week).
FIG. 17 is a representative AFM image according to an embodiment of the present invention (Aβ40/42 = 5/5 µM, 37°C, 2000 rpm, incubated in aCSF for 24 h).
FIG. 18 is a representative AFM image according to an embodiment of the present invention (Aβ40/42 = 5/5 µM, 37°C, 2000 rpm, incubated in aCSF for 1 week).
FIG. 19 is a representative example for monitoring Aβ aggregation kinetics via ThT according to an embodiment of the present invention, where incubation conditions for an Aβ aggregate are as follows: 25 µM of Aβ42 + 40 µM of ThT are incubated in DPBS at 37°C.
FIG. 20 shows an example of binding between ThT and Aβ aggregate according to an embodiment of the present invention, where an incubation condition for the Aβ aggregate is as follows: 5 µM of Aβ42 is incubated in DPBS at 37°C for 78 h, 40 µM of ThT.
FIG. 21 illustrates screening of a compound capable of inhibiting Aβ oligomerization by AFM according to an embodiment of the present invention, where an incubation condition for the Aβ aggregate is as follows: the Aβ aggregate is incubated in 10 mM HCl + 150 mM NaCl at 37°C for 48 h.
FIG. 22 illustrates screening of a compound capable of inhibiting Aβ oligomerization by AFM according to an embodiment of the present invention, where an incubation condition for the Aβ aggregate is as follows: the Aβ aggregate is incubated in DPBS at 37°C for 78 h.
FIG. 23 shows aggregation states before and after compound addition observed by AFM according to an embodiment of the present invention, where the left side shows 25 µM of a N3pE42 aggregate (DPBS, 70 h) and the right side shows 25 µM of a N3pE42 aggregate (DPBS, 70 h) + 40 µM of a compound 1', then incubated for 90 h.
FIG. 24 is a representative AFM image according to an embodiment of the present invention, Aβ38 (10 µM, 37°C, 2000 rpm, incubated in aCSF for 24 h).
FIG. 25 is a representative AFM image according to an embodiment of the present invention, Aβ38 (2 µM, 41°C, 2000 rpm, incubated in aCSF for 24 h).
FIG. 26 is a representative AFM image according to an embodiment of the present invention, Aβ38 (2 µM, 41°C, 2000 rpm, incubated in a Lysosome-mimicking fluid for 24 h).
FIG. 27 is a representative AFM image according to an embodiment of the present invention, Aβ42 (10 µM, 37°C, 0 rpm, incubated in HBS for 24 h).
FIG. 28 is a representative AFM image according to an embodiment of the present invention, Aβ40 (50 µM, 37°C, 0 rpm, incubated in HBS for 24 h).
FIG. 29 is a representative AFM image according to an embodiment of the present invention, Aβ40 (50 µM, 37°C, 0 rpm, incubated in HBS for 1 week).
FIG. 30 shows Aβ aggregation states observed by atomic force microscope (AFM) before and after the addition of the compound.
FIG. 31 shows effects of administration of different drugs on the area of Aβ plaques in mouse brains.
FIG. 32 shows effects of administration of different drugs on the area of activated microglia in mouse brains.
FIG. 33 shows effects of administration of different drugs on performance of mice in a water maze test.
FIG. 34 shows a comparison of [¹⁸F]AV45 uptake ratios in the hippocampus and cerebral cortex between mice in a treatment group and a model group.
FIG. 35 shows a comparison of [¹⁸F]DPA714 uptake ratios in the hippocampus and cerebral cortex between mice in a treatment group and a model group.

### DETAILED DESCRIPTION

Animal models used in the present application are as follows:
APP/PS1 mice (i.e., AD mice): purchased from The Jackson Laboratory, 6-month-old or 16-month-old, half male and half female.
C57BL/6J mice (i.e., normal control mice/wild mice): gender-matched with mice in the experimental group.

### Example 1 Synthesis of a compound represented by formula D

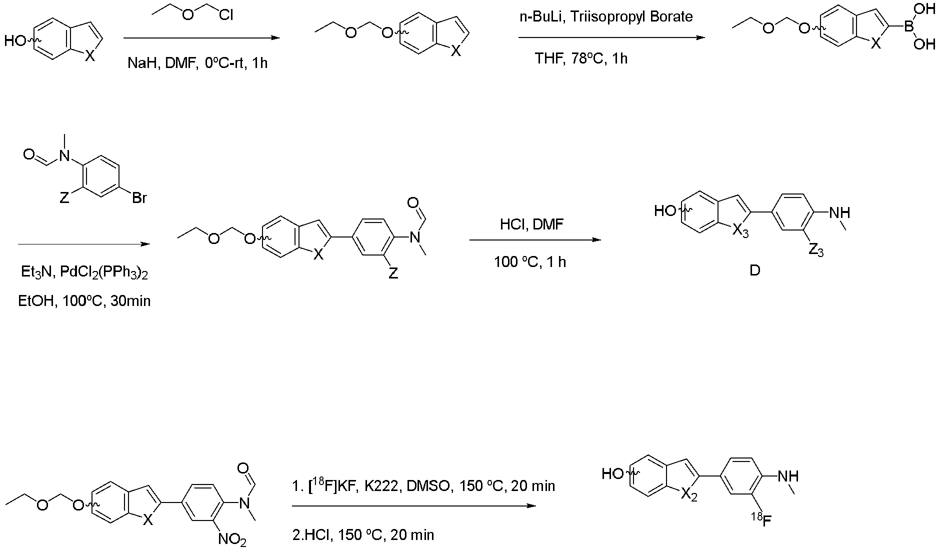

Taking 6-position hydroxyl substitution, X = O, Y = C and Z = F-18 as an example, other compounds in the general formula can be synthesized by the same method via substrate replacement.

### Step (1) Synthesis of 6-[(ethoxymethyl)oxy]-1-benzofuran

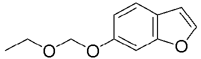

6-hydroxybenzofuran (2.7 g, 20.0 mmol) was dissolved in dried THF (30 mL), and after being cooled in an ice bath, NaH was added in batches (60% of NaH was dispersed in kerosene, 1.2 g, 30.0 mmol). After stirring for 10 min, chloromethyl ethyl ether (1.89 g, 20.0 mmol) was dropped into the above mixture, the mixture was stirred at room temperature for 3 h, the reaction was quenched with a saturated aqueous ammonium chloride solution, ethyl acetate was added for extraction, an organic layer was washed with a saturated NaCl solution and dried over anhydrous magnesium sulfate, after filtration, the solvent was removed under reduced pressure, and a crude product was purified by flash chromatography to give 6-[(ethoxymethyl)oxy]-1-benzofuran (2.9 g, yield: 75%).

### Step (2) Synthesis of {6-[(ethoxymethyl)oxy]-1-benzofuran-2-yl}boronic acid

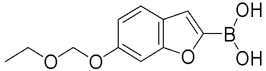

6-[(ethoxymethyl)oxy]-1-benzofuran (0.96 g, 5.0 mmol) was dissolved in a solution of dried THF (20 mL), after the mixture was cooled to -78°C, a 2.4 M n-butyllithium hexane solution (2.5 mL, 6.0 mmol) was slowly dropwise added. The mixture was stirred at -78°C for 1 h. Then, triisopropyl borate (1.4 mL, 6.0 mmol) was dropped into the mixture, and the mixture was continuously stirred at -78°C for 30 min. A reaction solution was slowly heated to room temperature and stirred for 30min. After the reaction was finished, 2 N HCl aqueous solution (6.0 mL) was dropwise added at room temperature, then the mixture was poured into water (20 mL), and extracted with ethyl acetate, an organic layer was washed with a saturated NaCl solution and dried over anhydrous magnesium sulfate, after filtration, a solvent was removed under reduced pressure, and a crude product was purified by flash chromatography to give a product 6-[(ethoxymethyl)oxy]-1-benzofuran-2-yl}boronic acid (0.35 g, yield: 30%).

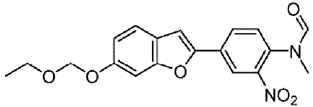

### Step (3) Synthesis of [(4-{6-[(ethoxymethyl)oxy]-1-benzofuran-2-yl}-2-nitrophenyl)(methyl)amino]2-methylpropan-2-yl methanoate

{6-[(ethoxymethyl)oxy]-1-benzofuran-2-yl}boronic acid (140 mg, 0.59 mmol) was dissolved in ethanol (10 mL), 2-methylpropan-2-yl [(4-bromo-2-nitrophenyl)(methyl)amino]formate (151 mg, 0.46 mmol), Pd(PPh₃)₂Cl₂(42 mg, 0.06 mmol) and Et₃N(127 µL, 0.91 mmol) were added. A reaction mixture was reacted in a microwave reactor at 100°C for 30 min. The mixture was concentrated under reduced pressure, a residue was diluted with water (15 mL), the mixture was extracted with ethyl acetate, an organic layer was washed with a saturated NaCl solution and dried over anhydrous magnesium sulfate. After filtration, a solvent was removed under reduced pressure, and a crude product was purified by flash chromatography to give N-(4-(6-(ethoxymethyl)benzofuran-2-yl)-2-nitrophenyl)-N-methylformamide (70 mg, yield: 41%); ESMS: m/z 371.12[M+1]⁺; 1H NMR(500MHz, Chloroform-*d*)δ 8.58(q, *J*=0.9 Hz,1H),8.40(d, *J*=2.1 Hz,1H),7.98(dd, *J*=7.4,2.1 Hz,1H),7.70-7.63(m, 1H),6.88-6.83(m, 1H),5.07(s, 1H),3.62(q, *J*=7.4 Hz, 1H),3.48(d, *J* = 1.0 Hz, 2H), 1.18(t, *J* = 7.5 Hz, 2H).

### Step (4) Synthesis of 2-(3-fluoro-4-(methylamino)phenyl)benzofuran-6-ol (preparation of the compound 1')

4.1, {6-[(ethoxymethyl)oxy]-1-benzofuran-2-yl}boronic acid (140 mg, 0.59 mmol) was dissolved in ethanol (10 mL), 2-methylpropan-2-yl [(4-bromo-2-fluorophenyl)(methyl)amino]formate (107 mg, 0.46 mmol), Pd(PPh₃)₂Cl₂(42 mg, 0.06 mmol) and Et₃N(127 µL, 0.91 mmol) were added. A reaction mixture was stirred in a microwave reactor at 100°C for 30 min. The mixture was concentrated under reduced pressure, a residue was diluted with water (15 mL), the mixture was extracted with ethyl acetate, an organic layer was washed with a saturated NaCl solution and dried over anhydrous sodium sulfate. After filtration, a solvent was removed under reduced pressure, and a crude product was purified by flash chromatography to give N-(4-(6-(ethoxymethyl)benzofuran-2-yl)-2-fluorophenyl)-N-methylformamide (90 mg, yield: 57%).

4.2, N-(4-(6-(ethoxymethyl)benzofuran-2-yl)-2-fluorophenyl)-N-methylformamide (90 mg, 0.26 mmol) was dissolved in DMF (5.0 mL)and 6 M HCl (5.0 mL) and stirred at 100°C for 60 min. Thin-layer chromatography (TLC) analysis indicated the completion of the reaction. The reaction was quenched by water, and the mixture was regulated with 1 N NaOH till the pH was neutral, extracted with dichloromethane and dried over anhydrous sodium sulfate. The mixture was concentrated under reduced pressure, and a crude product was purified by flash chromatography to give 2-(3-fluoro-4-(methylamino)phenyl)benzofuran-6-ol, which was a white solid (64 mg, yield: 96%). ESMS: m/z258.09[M+1]⁺;¹H NMR(500MHz, Chloroform-*d*)δ8.47(s,1H),7.43(dd, *J*=9.1,2.2Hz,2H),7.32(dd, *J*=12.1,2.2Hz,1H),7.00(dd, *J*=9.2,4.7Hz,1H), 6.93(d, *J*=2.0 Hz,1H), 6.85(d, *J*=2.3 Hz,1H), 6.78(dd, *J*=8.4 ,2.2Hz,1H), 6.63(m, *J*=4.6,3.5Hz,1H), 3.01(d, *J*=4.8 Hz,3H).

### Step (5) F-18 radiolabeling (the compound in the general formula A with X = O, the compound 1)

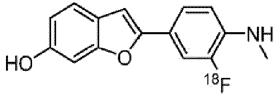

K2.2.2.(0.01 g, 25 mmol), potassium carbonate (12.5 mg) and acetonitrile (1 mL) were added to an aqueous solution of a [¹⁸F] fluoride (370-740 MBq), and the mixture was dried in a nitrogen flow at 110°C. The mixture was cooled to room temperature after being dried, and a DMSO (1.0 mL) solution of [(4-{6-[(ethoxymethyl)oxy]-1-benzofuran-2-yl}-2-nitrophenyl)(methyl)amino]2-methylpropan-2-yl methanoate (3 mg, 0.26 mmol) was added. The mixture was heated at 85°C for 15 min and cooled to 70°C, and hydrochloric acid (6 M, 0.25 mL) was added to continuously react for 30 min. Methanol/water (2 mL, V/V = 1/1) was added to dilute the reaction solution, the reaction solution was loaded onto a semi-preparative HPLC column (Luna 5 µm C18, 250*10 mm), and eluted with methanol/water (50: 50) at 4.0 mL/min. A product radioactive peak eluted at 18-20 min was collected and evaporated to dryness under nitrogen purge, and 0.5 mL of ethanol and 5.0 mL of a 0.9% NaCl solution were added to formulate the product substitute. The radiochemical yield ranged from 30% to 40%. The radiochemical purity (HPLC) was 97%.

### Example 2 Synthesis of a compound 2

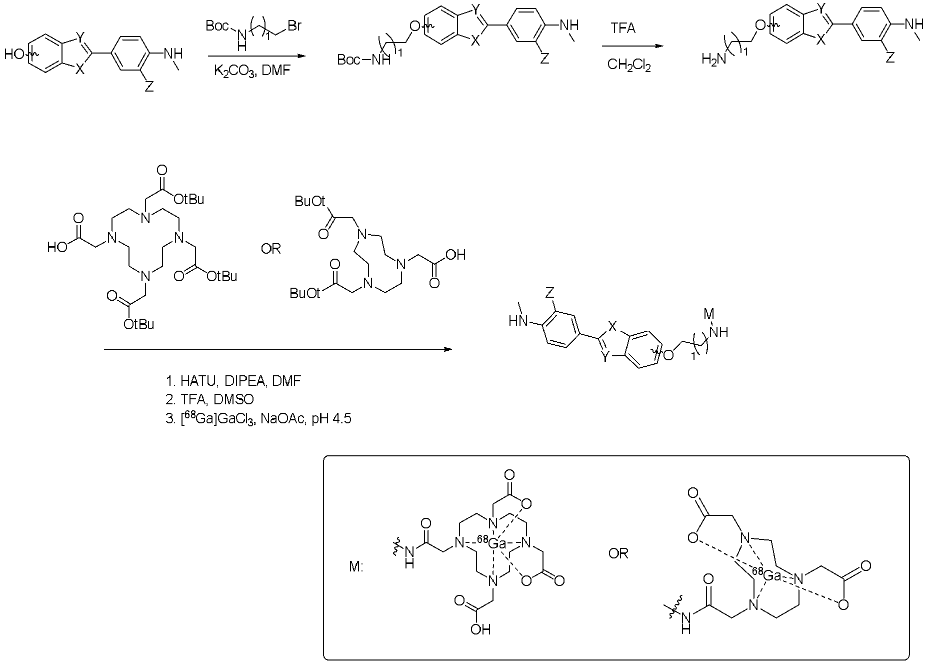

Taking 6-position hydroxyl substitution, X = S, Y = N, Z = H, n = 4, and a chelating group being DOTA as an example, other compounds in the general formula can be synthesized by the same method via substrate replacement.

### Step (1) Synthesis of tert-butyl (5-((2-(4-(methylamino)phenyl)benzo[d]thiazol-6-yl)oxy)pentyl)carbamate

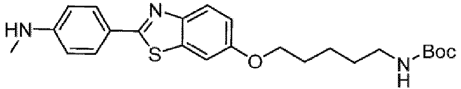

A mixture of 2-(4-(methylamino)phenyl)benzo[d]thiazol-6-ol (118 mg, 0.46 mmol), tert-butyl (5-bromopentyl)carbamate (245 mg, 0.92 mmol) and potassium carbonate (207 mg, 1.5 mmol) was dissolved in DMF (5 mL) and stirred at 90°C for 5 h. After the reaction was finished, the reaction solution was extracted with ethyl acetate and water, washed with saline, dried over anhydrous sodium sulfate, filtered, subjected to vacuum concentration, and silica gel column chromatography to give a pale yellow solid (175 mg, yield: 86%). LCMS (m/z): 442.2696[M+H]⁺. ¹HNMR (500MHz, DMSO) δ8.02-7.95(m,2H),7.91(d, J=8.9Hz,1H),7.81-7.75(m,1H),7.68(d,J=2.5Hz,1H),7.50-7.43(m,3H),7.11(dd,J=9.0,2.5Hz,1H),6.83-6.72(m, 1H), 4.03(q,J=7.2Hz,2H),3.99(t,J=6.3Hz,1H),3.25(s,3H),2.95(q,J=6.3Hz,2H),2.84(q,J=6.5Hz,1H), 2.42(s,2H),1.44(s,9H).

### Step (2) Synthesis of 4-(6-((5-aminopentyl)oxy)benzo[d]thiazol-2-yl)-N-methylaniline

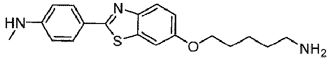

Tert-butyl (4-(6-hydroxybenzo[d]thiazol-2-yl)phenyl)(methyl)carbamate (170 mg, 0.5 mmol) and trifluoroacetic acid (1 mL) were dissolved in dichloromethane (1 mL), and the reaction solution was stirred overnight at room temperature. The reaction solution was concentrated under reduced pressure, and a residue was purified by semi-preparative HPLC to give a pale yellow solid (119 mg, yield: 70%). LCMS (m/z):342.1655[M+H]⁺.

### Step (3) Synthesis of tri-tert-butyl 2,2',2"-(10-(2-((5-((2-(4-(methylamino)phenyl)benzo[d]thiazol-6-yl)oxy)pentyl)amino)-2-oxoethyl)-1,4,7,10-tetraazacyclododecane-1,4,7-triyl)triacetate

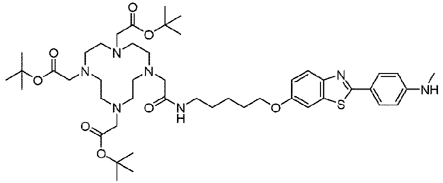

4-(6-((5-aminopentyl)oxy)benzo[d]thiazol-2-yl)-N-methylaniline (119 mg, 0.35 mmol), (10-{1-[(2-methylpropan-2-yl)oxy]-1-oxoethyl-2-yl}-4,7-bis{2-[(2-methylpropan-2-yl)oxy]-2-oxoethyl}-1,4,7,10-tetraazacyclododecan-1-yl)acetic acid (239 mg, 0.42 mmol) and an HBTU reagent O-(1H-benzotriazol-1-yl)-N,N,N',N'-tetramethylisouronium hexafluorophosphate (200 mg, 0.52 mmol) were dissolved in N,N-dimethylformamide (3 mL), and N,N-diisopropylethylamine (116 µL, 0.7 mmol) was added. The reaction solution was stirred overnight at room temperature. After the reaction was finished, the reaction solution was extracted with ethyl acetate and water, washed with saline, dried over anhydrous sodium sulfate, filtered, subjected to vacuum concentration, and silica gel column chromatography (dichloromethane: methanol = 20: 1) to give a pale yellow solid (214 mg, yield: 68%). LCMS(m/z): 896.5294[M+H]⁺. ¹HNMR(500MHz,CDCls) δ7.86-7.79(m,3H), 7.29(d,*J*=2.5Hz,1H), 6.98(dd,*J*=8.9,2.5Hz,1H), 6.67-6.61(m,2H), 6.31(t,*J*=5.9Hz,1H), 4.12(q,*J*=7.1Hz,4H), 3.99(t,*J*=6.5Hz,2H), 3.69(s,2H),3.22(q,*J*=6.7Hz,3H), 2.89(s,5H), 2.80(s,2H), 2.04(s,6H), 1.84-1.75(m,2H), 1.63-1.53(m,3H), 1.44(d,*J*=2.1Hz,27H), 1.26(t,*J*=7.1Hz,8H).

### Step (4) Synthesis of 2,2',2"-(10-(2-((5-((2-(4-(methylamino) phenyl)benzo[d]thiazol-6-yl)oxy)pentyl)amino)-2-oxoethyl)-1,4,7,10-tetraazacyclododecane-1,4,7-triyl)triacetic acid (compound 2')

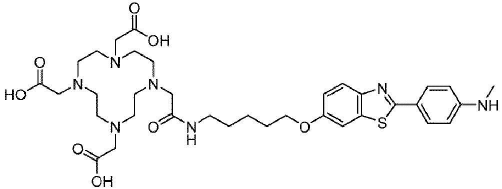

Tri-tert-butyl 2,2',2"-(10-(2-((5-((2-(4-(methylamino)phenyl)benzo[d]thiazol-6-yl)oxy)pentyl)amino)-2-oxoethyl)-1,4,7,10-tetraazacyclododecane-1,4,7-triyl)triacetate (170 mg, 0.18 mmol) and trifluoroacetic acid (1 mL) were dissolved in dichloromethane (1 mL), and the reaction solution was stirred overnight at room temperature. The reaction solution was concentrated under reduced pressure, and a residue was purified by semi-preparative HPLC to give a pale yellow solid (60 mg, yield: 46%). LCMS(m/z):728.3410[M+H]⁺. ¹HNMR(500MHz,DMSO)δ8.52(s,1H),7.83-7.73(m,3H),7.59(d, *J*=2.6 Hz,1H),7.04(dd, *J*=8.9 2.5Hz,1H),6.70-6.62(m,2H),4.60(s,4H),4.11(s,2H),4.04(t, *J*=6.4 Hz,2H),3.92(s,2H),3.56-3.35(m,8H),3.18-3.05(m,8H),2.76(s,3H),1.77(p, *J*=6.7 Hz,2H),1.56-1.43(m,4H),1.24(s,1H).

### Step (5) Synthesis of the compound 2

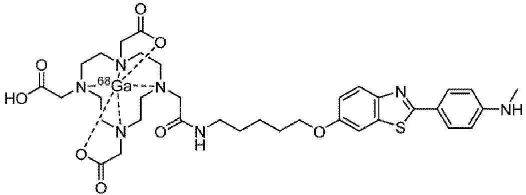

5.1 6 mL of a 0.6 M hydrochloric acid solution was drawn with a syringe, air bubbles were removed, then the hydrochloric acid solution was injected into a germanium-gallium generator to prepare eluent ⁶⁸GaCl₃. An eluate was collected in portions of 1 mL per tube, the radioactivity of each tube was measured, and the two tubes with the highest radioactivity were taken for subsequent labeling. (2 mL, about 20 mCi)

5.2 2 mL of ⁶⁸GaCl₃ obtained in 5.1 was taken, the pH value was regulated with a 3M NaOAc solution to about 4.0, 30 µL of a reaction precursor (the compound 2, 1.0 mg/mL), 10 µL of vitamin C, and 2.0 mL of acetonitrile were added, and the mixture was placed in a heater to react at 95°C for 15 min.

5.3 The reaction mixture obtained in the previous step was purified using a C18 solid-phase extraction cartridge.
(1) 10 mL of anhydrous ethanol was drawn with a syringe to activate the C18 cartridge.
(2) 10 mL of ultrapure water (or normal saline) was drawn with a syringe to rinse the C18 cartridge for later use.
(3) The labeling mixture was pushed slowly from Step 5.2 into the C18 cartridge with a syringe.
(4) 10 mL of ultrapure water (or normal saline) was drawn with a syringe and rinse the C18 cartridge three times.
(5) 0.2-0.3 mL of anhydrous ethanol was drawn with a syringe to elute the C18 cartridge, to obtain the ethanolic solution of radiolabeled product.

5.3 The ethanolic solution obtained in the previous step was diluted with normal saline until the ethanol concentration was less than 10%, and detection was performed by radio-high performance liquid chromatography. Chromatographic conditions are as follows:
Chromatographic column: Luna 5 µm, C18, 100Å, 250*4.6 mm

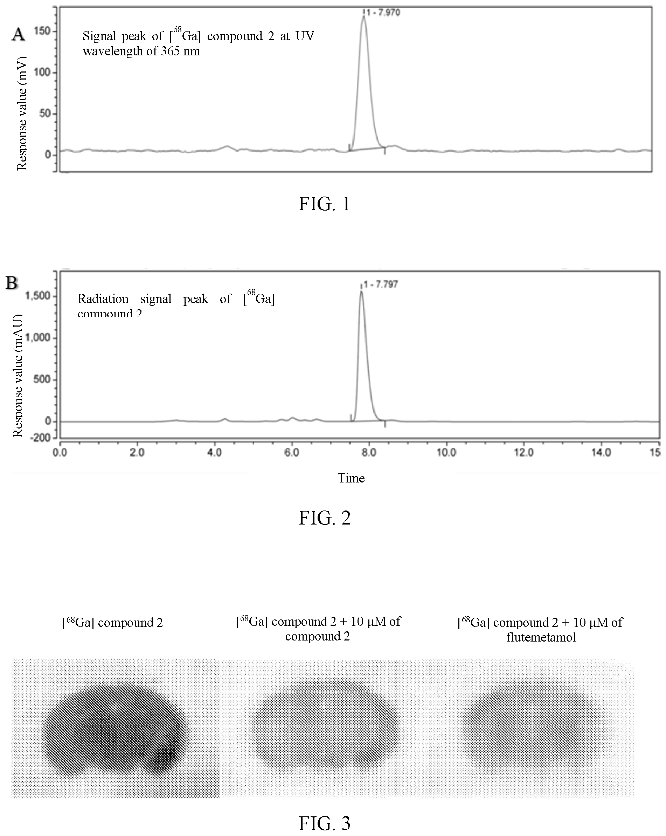

Mobile phase: A: MeCN containing 0.1% TFA; B: H₂O containing 0.1% TFA 0-2 min 20% A; 2-8 min, 20%A-95%A; 8-10 min 95%A; 10-12 min, 20%A. The retention time of the labeled product is 7.9 min, and the radiochemical purity is greater than 95% (see FIG. 1 and FIG. 2; the HPLC peak time of the compound 2 is consistent with that of the compound 2").

### Example 3 Synthesis of a compound 3

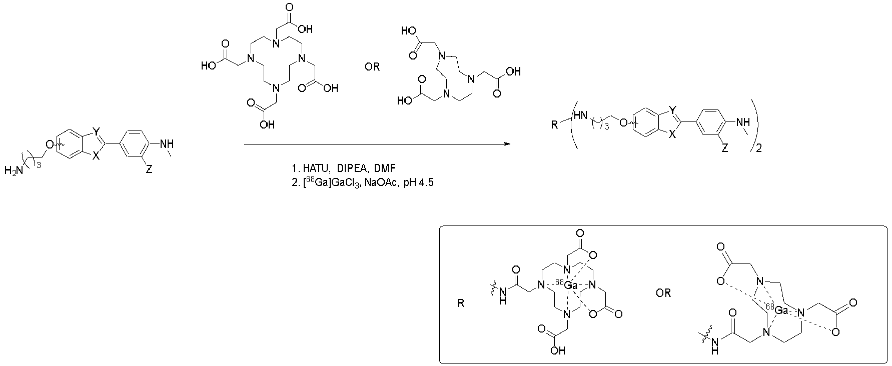

Taking 3-position hydroxyl substitution, X = S, Y = C, Z = H, n = 3, and a chelating group being NOTA as an example, other compounds in the general formula can be synthesized by the same method via substrate replacement.

### Step (1) Synthesis of 4-({2-[4-(methylamino)phenyl]benzo[b]thiophen-3-yl}oxy)butan-1-amine

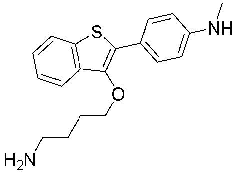

Tert-butyl (4-((2-(4-(methylamino)phenyl)benzo[b]thiophen-3-yl)oxy)butyl)carbamate (600 mg, 1.4 mmol) was dissolved in dichloromethane (2 mL) and trifluoroacetic acid (2 mL). A reaction mixture was stirred at room temperature for 3 h, and after the reaction was completed, a solvent was removed under reduced pressure to give a pale yellow oily compound (530 mg, yield: 86%), which is directly used for a next reaction.

### Step (2) Synthesis of [4,7-Bis(2-{[4-({2-[4-(methylamino)phenyl] benzo[b]thiophen-3-yl}oxy)butyl]amino}-2-oxoethyl)-1,4,7-triazonan-1-yl]acetic acid (compound 3')

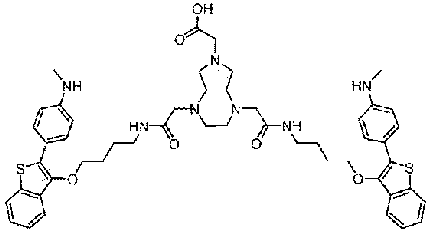

2,2',2"-(1,4,7-triazonane-1,4,7-triyl)triacetic acid (60 mg, 0.2 mmol) was dissolved in DMSO (1 mL), and 4-({2-[4-(methylamino)phenyl]benzo[b]thiophen-3-yl}oxy)butan-1-amine (130 mg, 0.4 mmol), EDC (62 mg, 0.4 mmol), HOBt (54 mg, 0.4 mmol) and DIPEA (104 mg, 0.8 mmol) were added. The mixture was stirred at room temperature for 1 h, and a reaction progress was monitored by high performance liquid chromatography-mass spectrometry. A reaction product was purified by preparative HPLC. A C18 reversed-phase chromatographic column (10×250 mm, 5 µm) was used at a flow rate of 4.0 mL/min. A linear gradient of 20% to 80% solvent B was maintained for 20 min, followed by a linear gradient of 80% to 100% solvent B for 5 min, and finally isocratic elution with 100% solvent B for another 5 min. Solvent A was an aqueous solution containing 0.1% trifluoroacetic acid, and solvent B was an acetonitrile solution containing 0.1% trifluoroacetic acid. Corresponding product peaks were collected, a solvent was removed at reduced pressure, and the mixture was lyophilized to give a white solid product (55 mg, yield: 15%). ESMSm/z: 920.41(M+1)⁺;¹HNMR (500MHz,DMSO-d₆)δ: 9.71(s,1H),8.36(s,2H),7.82(s,2H),7.62(d,J=7.9Hz,2H),7.56(s,4H), 7.35(s,2H),7.28(t,J=7.5Hz,2H),6.63(s,4H),6.08(s,2H),3.85(t,J=6.3Hz,4H),3.53(s,2H),3.22(s,4 H),3.11(s,8H),2.83(s,4H),2.68(s,7H),2.60(s,4H), 1.68(s,4H), 1.60(s,4H).

### Step (3) Synthesis of a compound 3

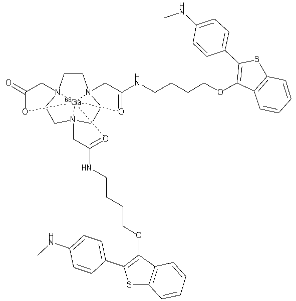

A ⁶⁸GaCl₃ solution (1 mL) was taken, the pH value was regulated with a 3M NaOAc solution to about 4.0, 30 µL of a labeling precursor (1.0 mg/mL), 10 µL of vitamin C, and acetonitrile (1.0 mL) were added. The mixture was placed in a heater to react at 95°C for 15 min. Purified via the C18 cartridge, the mixture was eluted with ethanol to obtain a solution of ⁶⁸Ga-labeled probe. The preparation method refers to Step (4) of Example 3.

### Example 4 Synthesis of a compound 4

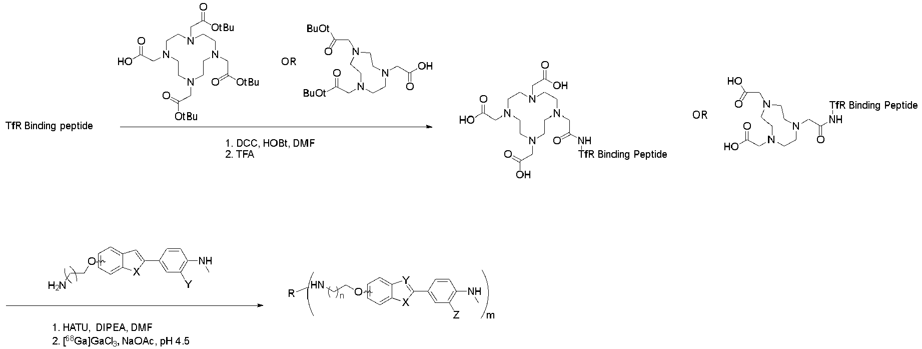

Taking 6-position hydroxyl substitution, X = S, Y = N, Z = H, n = 4, and a chelating group being DOTA modified with THRPPMWSPVWP as an example, other compounds in the general formula can be synthesized by the same method via substrate replacement.

### Step (1) Synthesis of DOTA-THRPPMWSPVWP

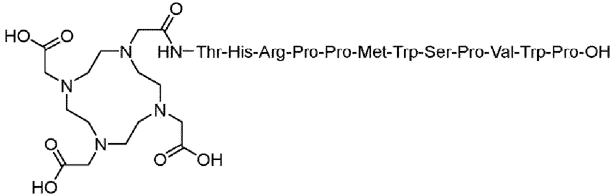

2-(4,7,10-tris(2-(tert-butoxy)-2-oxoethyl)-1,4,7,10-tetraazacyclododecan-1-yl)acetic acid (21 mg, 60 µM) and HBTU (O-(benzotriazol-1-yl)-N,N,N',N'-tetramethyluronium hexafluorophosphate) (23 mg, 60 µM) were dissolved in N,N-dimethylformamide (1.0 mL), N,N-diisopropylethylamine (7.8 mg, 60 µM) was added, and the mixture was stirred at room temperature for 2 min. A reaction mixture was added to THRPPMWSPVWP (22 mg, 15 µM), and was reacted for 3 h. The reaction completion was monitored by HPLC. Trifluoroacetic acid (1.0 mL) was added, and the mixture was stirred at room temperature for 8 h. After the reaction finished, the reaction solution was purified by semi-preparative HPLC with a linear gradient of 0-100% MeOH + 0.1% TFA within 30 min. A product was isolated as a white solid after lyophilization (14 mg, yield: 48%). ESI-MS(m/z) for [M+1]⁺ (calculated):1892.6(1892.19).

### Step (2) Synthesis of a DOTA diligand compound (compound 4')

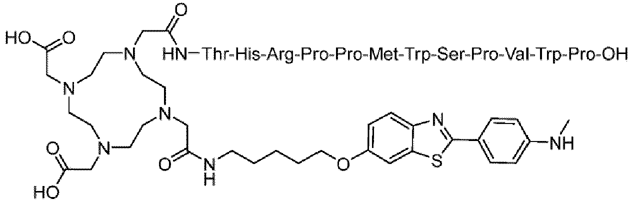

DOTA-THRPPMWSPVWP (14 mg, 7.2 µM) and HBTU (O-(benzotriazol-1-yl)-N,N,N',N'-tetramethyluronium hexafluorophosphate (4 mg, 10 µM) were dissolved in N,N-dimethylformamide (1.0 mL), N,N-diisopropylethylamine (2 mg, 10 µM) was added, and the mixture was stirred at room temperature for 2 min. 4-(6-((5-aminopentyl)oxy)benzo[d]thiazol-2-yl)-N-methylaniline (synthesized by general formula B, step 2) (1.0 mg, 3µM) was added to a reaction mixture and was reacted for 8 h. The reaction was monitored by HPLC. A reaction solution was purified by semi-preparative HPLC using a gradient of 0-100% MeOH + 0.1% TFA within 30 min. After lyophilization, the compound was isolated as a white solid (2 mg, yield: 30%). ESI-MS(m/z)for [M+1]⁺(calculated):2216.6(2216.34).

### Step (3) Synthesis of a ⁶⁸Ga label of a DOTA-diligand compound (compound 4)

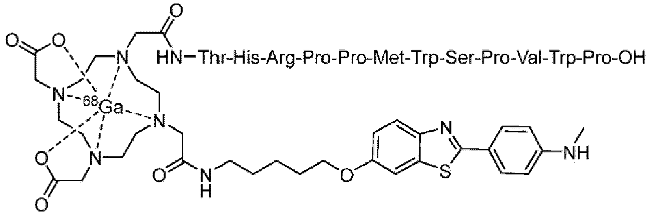

A ⁶⁸GaCl₃ solution (1 mL) was taken, the pH value was regulated with a 3 M NaOAc solution to about 4.0, 30 µL of a labeling precursor (1.0 mg/mL) and 10 µg of vitamin C were added, and the mixture was placed in a heater at 80°C for 15 min, purified via a C18 cartridge, and eluted with ethanol to give a ⁶⁸Ga-labelled probe solution. The preparation method refers to synthesis step (4) of general formula B.

### Example 5 In vitro Aβ binding affinity assay of the compound to Aβ

### 1) Determination of in vitro Aβ binding affinity of the reference compound via SPR

A Biacore 8K surface plasmon resonance instrument was used to analyze the interaction between the compound and Aβ. A system temperature was set at 25°C and a flow rate at 10 µL/min. A flow cell 2 (FC-2) was set as a protein coupling channel, and a flow cell 1 (FC-1) was set as a reference channel to detect non-specific binding and subtract background signals.

Selection of preconditioning-coupling pH value: when a chip surface was not activated, an Aβ sample (GL Biochem, Cat#52487) was dissolved in sodium acetate buffers with different pH values (pH = 4.0, 4.5, 5.0, 5.5), such that the sample carried different amounts of positive charges, then a protein sample flowed over the chip surface at a flow rate of 10 µL/min, and the optimal pH condition was determined according to the binding curve between integrin and the chip.

Coupling of Aβ: a CM5 chip was selected. Coupling of the protein was operated in accordance with an amine coupling method. The coupling steps were as follows: (1) activation: a NHS/EDC mixture was injected at a flow rate of 10 µL/min (0.1 M NHS and 0.4 M EDC were mixed at a volume ratio of 1: 1 before use, and were immediately used) to activate carboxyl of dextran on the surface of the activation chip; (2) coupling: 50 µg/mL Aβ solutions (Aβ42 monomer, Aβ42 oligomer and Aβ42 fibril) using buffers with pH values determined in the pre-treatment process. The preparation methods of Aβ42 oligomer and Aβ42 fibril refer to Alzheimer's disease and Frontotemporal Dementia, 2010, 670, 13-32. The solutions were injected at a flow rate of 10 µL/min until the immobilization level reached approximately 3000 RU; (3) Blocking: Blocking was performed with 1 M ethanolamine hydrochloride to complete the protein coupling process.

In a binding assay, an analyte to be tested was dissolved in DMSO to prepare a 10 mM stock solution, which was then diluted with PBS-P+ (Cytiva) to obtain a series of required concentrations. A sample was injected at a flow rate of 30 µL/min for 5 min, with the association phase (kₒₙ) lasting for 60 s and the dissociation phase (k_{off}) lasting for 60 s. Experimental results were analyzed after solvent correction, and the sensorgram of reference channel was subtracted from that of sample channel. The concentrations of the analytes were respectively 3.91 µM, 7.81 µM, 15.63 µM, 31.25 µM, 62.50 µM, and 125.0 µM. The kinetic parameters were obtained by simulation based on the assumed 1: 1 binding model.

In the SPR experiment, when the analyte solution flowed over the chip surface, the analyte bound to the protein conjugate immobilized thereon, causing changes in surface mass of the chip and further resulting in variation of resonance signals. The interaction results thereof were presented in sensorgrams, where the ordinate represented the binding level between small molecules and proteins, which was continuously recorded as response units (RU), and the abscissa denoted time. The dissociation equilibrium constant (KD) was obtained by fitting, and the experimental results of partial compounds are shown in Table 1.

**Table 1 Results of the binding abilities of the compounds to Aβ**

| Compounds | KD(M) | | |
|---|---|---|---|
| | Aβ42 monomer | Aβ42 oligomer | Aβ42 fibril |
| Compound 1' | 5.60×10⁻⁶ | 8.42×10⁻⁶ | 6.21×10⁻⁶ |
| Compound 2' | 8.62×10⁻⁶ | 4.71×10⁻⁶ | 4.25×10⁻⁶ |
| Compound 3' | 5.74×10⁻⁵ | 2.41×10⁻⁵ | 2.96×10⁻⁴ |
| Compound 4' | NA | NA | NA |
| Flutemetamol (control sample 1) | 3.36×10⁻⁶ | 7.31×10⁻⁷ | 2.24×10⁻⁶ |
| AZD4694 (i.e., Flutafuranol) (control sample 2) | 3.33×10⁻⁶ | 5.82×10⁻⁶ | 3.28×10⁻⁶ |

The results show that the exemplary compound 1' and compound 2' provided in the present application exhibit favorable Aβ binding capacity, and in SPR assays, exhibit binding capacities equivalent to those of reference compounds Flutemetamol and AZD4694 (i.e., Flutafuranol) against Aβ42 monomers, Aβ42 oligomers and Aβ42 fibrils.

2) The compound competes with Thioflavin T (ThT) for binding to Aβ aggregates.

The Aβ aggregate (including various forms such as fibrils, plaques, and oligomers) prepared in step 1) was added to a 96-well plate, under neutral conditions, DMSO solution containing 1 mM standard compound and an equal volume of DMSO free of standard compound were added respectively, where the Aβ concentration was 5 µM, the standard compound concentration was 40 µM, a fluorescence value (Ex/Em: 440 nm/490 nm) was measured at 37°C using a fluorescence spectrophotometer (BioTek, Cytation 3), and subsequently, the fluorescence value (Ex/Em: 440 nm/490 nm) was measured after ThT (a final concentration was 40 µM) was added to each well. Three parallel replicates were set for each group, and statistical analysis was performed via GraphPad Prism.

In the Thioflavin T (ThT) competitive binding assay, the exemplary compound 1' and compound 2' compete with Thioflavin T (ThT) for binding to the Aβ aggregates. The compounds themselves show no obvious fluorescence under the determination conditions. After incubation of the compounds with the Aβ aggregates followed by addition of ThT, the fluorescence intensity is significantly lower than that of the control group without compound addition, indicating that the compound 1' and compound 2' can competitively bind to the Aβ aggregates with ThT.

| Experimental subjects Fluorescence distribution | Aβ | Aβ + ThT | Aβ + compound 1' | Aβ + compound 1' + ThT | Aβ + compound 2' | Aβ + compound 2' + ThT |
|---|---|---|---|---|---|---|
| Mean fluorescence intensity | 1834 | 5731 | 1790 | 2477 | 2392 | 2485 |
| Standard deviation | 12 | 794 | 20 | 61 | 268 | 11 |

### Example 6 Autoradiography experiment of a Ga-68-labeled Aβ PET imaging agent (compound 2) in a transgenic AD mouse (APP/PS1) model

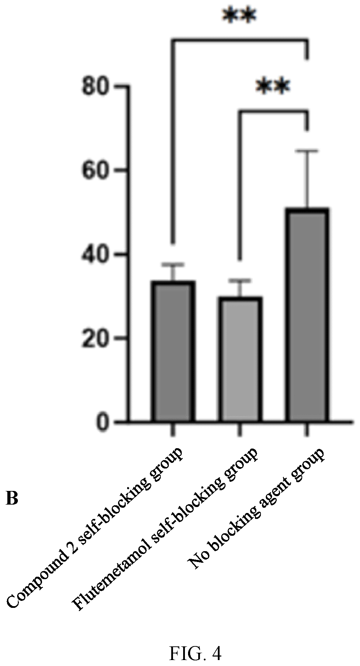

APP/PS1 mice (purchased from The Jackson Laboratory, 16-month-old, half male and half female) and age-matched wild-type mice were anesthetized with isoflurane, and whole brains were dissected and isolated. The whole brains were embedded with OCT embedding medium to prepare frozen sections (-20 °C, 10 µm). Surfaces of the brain sections were first covered with PBS (pH 7.4) and pre-incubated at room temperature for 10 min to eliminate endogenous binding. The surfaces of the brain sections were first covered with the solution (about 60 µCi/mL) of the compound 2 prepared in step 5.2 in example 2 and incubated at room temperature for 60 min. The positive compound Flutemetamol (10 µM) and the unlabeled compound 2 (10 µM) were added to the incubation solution for the Block group and Self-Block group, respectively. After incubation, the sections were rinsed twice with PBS (pH 7.4) for 2 min each time, followed by two washes with 40% aqueous ethanol solution for 2 min each time, and finally rinsed with deionized water for 10 s, then air-dried at room temperature. The sections were covered with plastic wrap and exposed to a phosphor screen at room temperature for 12 h. A phosphor screen was scanned using a phosphor imaging system (Typhoon FLA9500, GE Healthcare) in a dark condition with a scanning resolution of 25 µm. A quantitative analysis of autoradiographic images was performed using image analysis software (Image Quant TL, GE Healthcare) to evaluate the specific binding ability of the probe in mouse brain tissues. The results are shown in FIG. 3 and FIG. 4. Experimental results showed that the compound 2 could specifically bind to Aβ in the brains of APP/PS1 mice, and such binding signals could be blocked by the known Aβ binder flutemetamol and the compound 2.

### Example 7 PET Imaging of the compound 2 in a transgenic AD mouse (APP/PS1) model

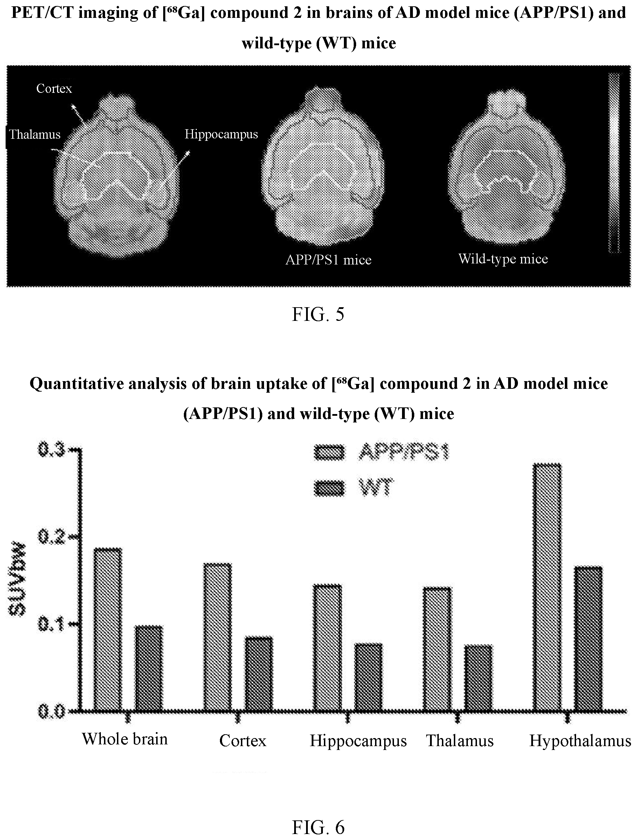

One day prior to the experiment, mice in the control group and the APP/PS1 model group were fasted with free access to water for no more than 18 h (each group contained 8 mice, with a total of 16 mice). The mice were placed in an anesthesia chamber for isoflurane inhalation anesthesia, and transferred to an animal bed of the instrument after full anesthesia. Under isoflurane anesthesia, the PET molecular probe solution (approximately 500 µCi, 0.1 mL) prepared in step 5.2 of example 2 was injected into mice via tail vein, followed by PET/CT imaging for approximately 0-60 minutes as required, with inhalation anesthesia maintained throughout the imaging process. After imaging, the small animals were removed from the instrument and allowed to wake up naturally. The experimental results showed that the uptake of the compound 2 in the brains of APP/PS1 AD model mice was higher than that in the control group (FIG. 5 and FIG. 6), which was consistent with the results of in vitro autoradiography.

The success rate of anti-Aβ drug development is relatively low in the prior art. The inventors have unexpectedly found that the morphology, particle size and aggregation state of target Aβ aggregates exert influences on the efficiency of screening methods and the efficacy of screened drugs. In the prior art, the incubation conditions for preparing Aβ aggregates vary greatly and differ substantially from the in vivo microenvironment, which is considered by the inventors to be one of the key factors leading to the low success rate of anti-Aβ drug research and development. Further, the inventors prepared different Aβ aggregates by simulating different microenvironments in the human brain, including: simulating a buffer solution in the internal environment of endosomes/lysosomes, simulating normal human body temperature, temperature of superficial brain regions and temperature of deep brain regions at different temperatures of 37°C, 38.5°C, and 41°C, and simulating various different conditions such as human static state, exercise state and blood flow effects under different rotational speeds of 0-2000 rpm. Under the combined above conditions, single-sequence Aβ aggregates were incubated and prepared for specific binding screening. Meanwhile, aggregates formed by co-incubating Aβ40 with other Aβ sequences were prepared to simulate complex endogenous Aβ aggregates in vivo for general screening.

### Method for preparing an Aβ aggregate

The present invention relates to a method for preparing an Aβ aggregate, including the following steps: S1, dissolving an Aβ lyophilized powder in hexafluoroisopropanol and drying to remove hexafluoroisopropanol to obtain an Aβ peptide film; S2, mixing the Aβ peptide film with a buffer solution to obtain an Aβ solution with a concentration of 0.2-100 µM; and S3, incubating the Aβ solution to obtain an Aβ aggregate.

The Aβ lyophilized powder can be selected from one or more Aβ isoforms such as Aβ40, Aβ42, Aβ38, N3pE42, or a combination thereof. These isoforms are all known in sequence and commercially available. For example, the Aβ aggregate is an Aβ40 oligomer, an Aβ42 oligomer or an oligomer formed by any two or all of Aβ40, Aβ42, Aβ38, and N3pE42. When various Aβ isoforms are mixed and incubated, preferably, at least one isoform is Aβ40, and more preferably, the Aβ40 is used as a main body. In some implementations of the present invention, the mixture of Aβ40 and other Aβ species are co-incubated to simulate diverse physiological and pathological states in the brain, for example, a mixture of Aβ40 and Aβ42, a mixture of Aβ40 and N3pE42, a mixture of Aβ40 and Aβ38, or a mixture of Aβ40, Aβ42, and N3pE42. When various Aβ isoforms are mixed and incubated, preferably, the molar percentage content of Aβ 40 is not less than 50%. In some implementations of the present invention, the molar ratio of Aβ40 and other Aβ is (5-9): (1-5), for example, Aβ40/42(9: 1), Aβ40/42(8: 2), Aβ40/42(7: 3), Aβ40/42(6: 4), Aβ40/42(5: 5), Aβ40/N3pE42(9: 1), Aβ40/42/N3pE42(5: 4: 1), Aβ40/42/N3pE42(5: 4: 1), and the like (values in parentheses are molar ratios).

The buffer solution is optionally a buffer solution that simulates different microenvironments in the human brain, and is preferably at least one of calcium- and magnesium-free Dulbecco's phosphate-buffered saline (DPBS), an HBS buffer solution containing 8-12 mM HEPES and 140-160 mM NaCl, artificial cerebrospinal fluid (aCSF), DMEM, FBS, endosome-mimicking fluid, and lysosome-mimicking fluid. In some implementations of the present invention, calcium- and magnesium-free Dulbecco's phosphate-buffered saline (DPBS), the artificial cerebrospinal fluid (aCSF), or the HBS buffer solution containing 8-12 mM HEPES and 140-160 mM NaCl is used as the buffer solution.

As incubation conditions, the incubation temperature is 36 to 41°C, preferably 37°C, 38.5°C, and 41°C, which respectively simulate the normal human body temperature, temperature of superficial brain regions and temperature of deep brain regions. The incubation shaking speed is 0 to 2500 rpm. For instance, 0 rpm and 2000 rpm are adopted to simulate static and motion states, respectively.

In the present invention, the term "Aβ aggregate" refers to an oligomer formed by aggregation of one or more amyloid-β monomers.

### Use of the Aβ aggregate

The present invention further relates to use of the Aβ aggregate prepared by the above method, as an anti-Aβ drug screening target to screen a drug. Due to the polymorphism of Aβ aggregates, the Aβ aggregates prepared by different methods are substantially distinct and serve as different targets. Compared with the Aβ aggregates prepared by conventional literature, those obtained by the method of the present invention enable more intuitive and rapid screening of effective candidate drugs. AFM images verify that the screened drugs exert disaggregation effect on the Aβ aggregates, which is a property of candidate drugs that cannot be directly determined by other conventional methods.

### A target for screening the anti-Aβ drug

The present invention further relates to a target for screening the anti-Aβ drug, where the target is the Aβ aggregate prepared by the above method. It can be observed from representative AFM images that the Aβ aggregates obtained in the present invention are obviously different from typical Aβ monomers, typical Aβ oligomers, typical Aβ fibrils and typical Aβ plaques, and belongs to distinct targets. The terms "typical Aβ monomer", "typical Aβ oligomer", "typical Aβ fibril" and "typical Aβ plaque" refer to typical Aβ monomer , typical Aβ oligomer , typical Aβ fibril and typical Aβ plaque prepared by the methods described in the comparative examples of the present invention, i.e., conventional methods disclosed in classic literature.

### A method for screening the anti-Aβ drug

The present invention further relates to a method for screening the anti-Aβ drug, including the following step: screening the anti-Aβ drug using the target for screening the anti-Aβ drug. Preferably, a drug capable of disaggregating the Aβ aggregate or binding to the Aβ aggregate or inhibiting aggregation of the Aβ aggregate is screened. There is no limitation on the type of the foregoing drug, which may be optionally selected from an antibody, a polypeptide, a small-molecule compound and the like.

### The anti-Aβ drug

The present invention further relates to an anti-Aβ drug screened by the method provided by the present invention, where the anti-Aβ drug is shown in the following formula I:

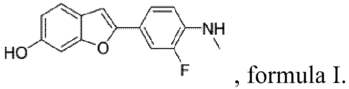

The present invention further confirms via the SPR assay and the ThT competitive binding assay that the compounds represented by formula I are capable of binding to Aβ, with binding affinity comparable to that of Flutemetamol and AZD4694 (i.e., Flutafuranol).

The present invention further relates to use of the compound represented by formula I for in vitro non-therapeutic use, (i) inhibiting formation of Aβ oligomers; (ii) promoting disaggregation of Aβ oligomers; and/or (iii) binding to Aβ oligomers.

In preferred implementations, the term "anti-Aβ drug" refers to a drug capable of disaggregating Aβ oligomers or inhibiting the formation of Aβ oligomers.

### Example 8

In this example, Aβ oligomers were prepared. A specific method includes the following steps:
(1) Preparation and storage of an Aβ peptide film: Hexafluoroisopropanol (HFIP) was added to artificially synthesized Aβ lyophilized powders Aβ40, Aβ42, and Aβ38 (purchased from Gill Biochemical) to prepare 1 mM solutions, the solutions were incubated at room temperature for 30 min, the solutions were aliquoted into 0.5 mL low-adhesion microcentrifuge tubes at 10 µL per tube, the microcentrifuge tubes were placed in a fume hood overnight to allow HFIP to volatilize and then transferred to a vacuum centrifugal device (SpeedVac) to be dried for 1 h to remove residual HFIP and moisture to obtain an Aβ peptide film, and the Aβ peptide film was sealed together with a desiccant and stored at -20 °C for later use.
(2) Preparation of an Aβ DMSO stock solution (i.e., an Aβ stock solution)
   The Aβ peptide film was taken out and equilibrated to room temperature, anhydrous DMSO (dimethyl sulfoxide) was added to prepare a 5 mM stock solution, the mixture was vortexed for 30 s, followed by ultrasonication for 10 min, and the stock solution was used as an initial material for preparing the Aβ aggregates.
(3) Preparation of single-sequence Aβ aggregates under conditions close to real in vivo environments

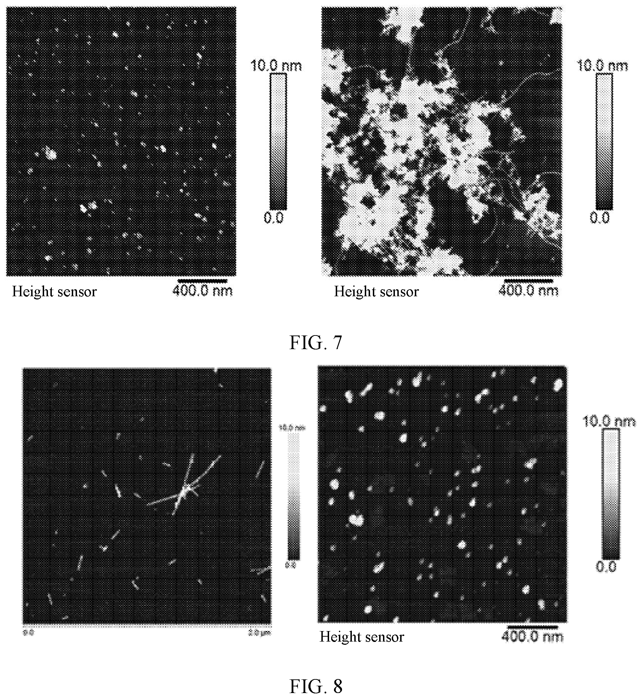

The Aβ DMSO stock solution with 5 mM Aβ prepared in (2) was taken, HBS was added to prepare a 100 µM Aβ solution, the solution was then diluted with an HBS buffer solution to 50 µM, incubated at 2000 rpm and 37 °C, and used after incubation for 24 h. The aggregation morphology was determined by AFM to obtain Aβ aggregates with corresponding aggregation degree and morphology. FIG. 8 is an AFM morphology image of Aβ40 and Aβ42.

### Example 9

The method for preparing the Aβ oligomer in this embodiment is substantially the same as that in example 8, with partial adjustments made to the incubation conditions.

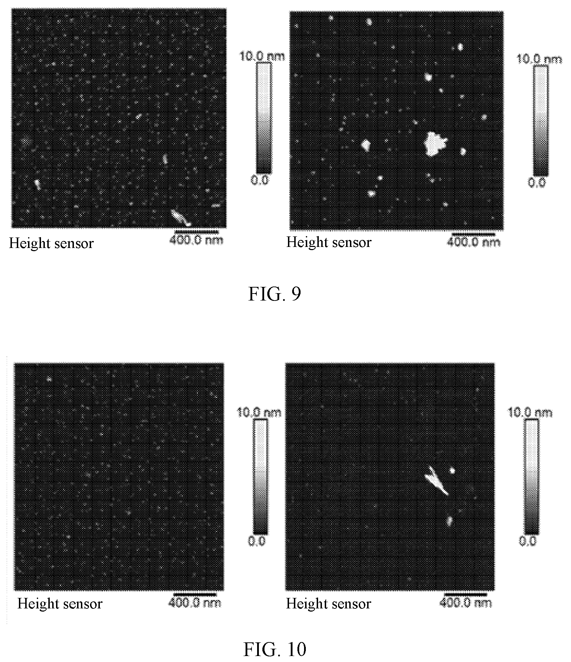

The Aβ DMSO stock solution with 5 mM Aβ prepared in (2) was taken, HBS was added to prepare a 100 µM Aβ solution, the solution was then diluted with an HBS buffer solution to 10 µM, incubated at 2000 rpm and 37°C, and used after incubation for 24 h. The aggregation morphology was determined by AFM to obtain Aβ aggregates with corresponding aggregation degree and morphology. FIG. 9 is an AFM morphology image of Aβ40 and Aβ42.

### Example 10

The method for preparing the Aβ oligomer in this embodiment is substantially the same as that in example 8, with partial adjustments made to the incubation conditions.

The Aβ DMSO stock solution with 5 mM Aβ prepared in (2) was taken, HBS was added to prepare a 100 µM Aβ solution, the solution was then diluted with an HBS buffer solution to 2 µM, incubated at 2000 rpm and 37°C, and used after incubation for 24 h. The aggregation morphology was determined by AFM to obtain Aβ aggregates with corresponding aggregation degree and morphology. FIG. 10 is an AFM morphology image of Aβ40 and Aβ42.

### Example 11

The method for preparing the Aβ oligomer in this embodiment is substantially the same as that in example 8, with partial adjustments made to the incubation conditions.

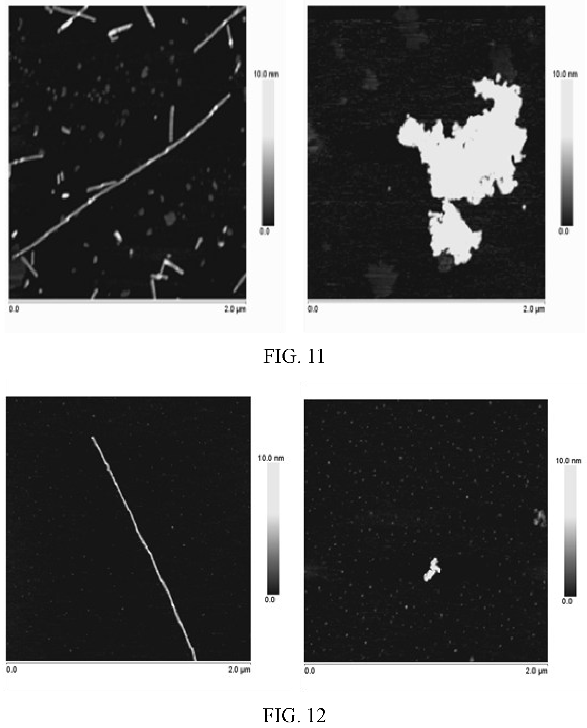

The Aβ DMSO stock solution with 5 mM Aβ prepared in (2) was taken, HBS was added to prepare a 100 µM Aβ solution, the solution was then diluted with an HBS buffer solution to 50 µM, incubated at 2000 rpm and 37°C, and used after incubation for one week. The aggregation morphology was determined by AFM to obtain Aβ aggregates with corresponding aggregation degree and morphology. FIG. 11 is an AFM morphology image of Aβ40 and Aβ42.

### Example 12

The method for preparing the Aβ oligomer in this embodiment is substantially the same as that in example 8, with partial adjustments made to the incubation conditions.

The DMSO stock solution with 5 mM Aβ prepared in (2) was taken, aCSF was added to prepare a 100 µM Aβ solution, the solution was then diluted with an aCSF buffer solution to 50 µM, incubated at 2000 rpm and 37°C, and used after incubation for 24 h. The aggregation morphology was determined by AFM to obtain Aβ aggregates with corresponding aggregation degree and morphology. FIG. 12 is an AFM morphology image of Aβ40 and Aβ42.

### Example 13

The method for preparing the Aβ oligomer in this embodiment is substantially the same as that in example 8, with partial adjustments made to the incubation conditions.

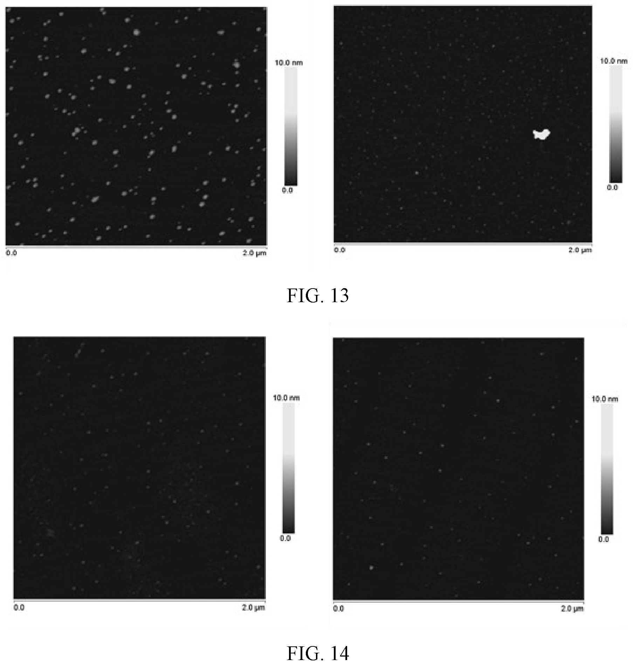

The DMSO stock solution with 5 mM Aβ prepared in (2) was taken, aCSF was added to prepare a 100 µM Aβ solution, the solution was then diluted with an aCSF buffer solution to 10 µM, incubated at 2000 rpm and 37°C, and used after incubation for 24 h. The aggregation morphology was determined by AFM to obtain Aβ aggregates with corresponding aggregation degree and morphology. FIG. 13 is an AFM morphology image of Aβ40 and Aβ42.

### Example 14

The method for preparing the Aβ oligomer in this embodiment is substantially the same as that in example 8, with partial adjustments made to the incubation conditions.

The DMSO stock solution with 5 mM Aβ prepared in (2) was taken, aCSF was added to prepare a 100 µM Aβ solution, the solution was then diluted with an aCSF buffer solution to 2 µM, incubated at 2000 rpm and 37°C, and used after incubation for 24 h. The aggregation morphology was determined by AFM to obtain Aβ aggregates with corresponding aggregation degree and morphology. FIG. 14 is an AFM morphology image of Aβ40 and Aβ42.

### Example 15

The method for preparing the Aβ oligomer in this embodiment is substantially the same as that in example 8, with partial adjustments made to the incubation conditions.

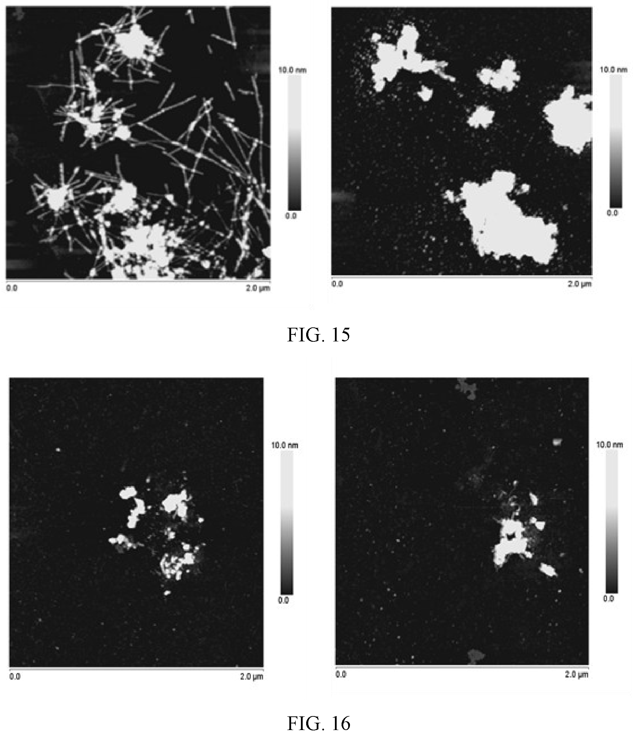

The DMSO stock solution with 5 mM Aβ prepared in (2) was taken, aCSF was added to prepare a 100 µM Aβ solution, the solution was then diluted with an aCSF buffer solution to 50 µM, incubated at 2000 rpm and 37°C, and used after incubation for 1 week. The aggregation morphology was determined by AFM to obtain Aβ aggregates with corresponding aggregation degree and morphology. FIG. 15 is an AFM morphology image of Aβ40 and Aβ42.

### Example 16

The method for preparing the Aβ oligomer in this embodiment is substantially the same as that in example 8, with partial adjustments made to the incubation conditions.

The DMSO stock solution with 5 mM Aβ prepared in (2) was taken, aCSF was added to prepare a 100 µM Aβ solution, the solution was then diluted with an aCSF buffer solution to 10 µM, incubated at 2000 rpm and 37°C, and used after incubation for 1 week. The aggregation morphology was determined by AFM to obtain Aβ aggregates with corresponding aggregation degree and morphology. FIG. 16 is an AFM morphology image of Aβ40 and Aβ42.

### Example 17

The method for preparing the Aβ oligomer in this embodiment is substantially the same as that in example 8, with partial adjustments made to the incubation conditions.

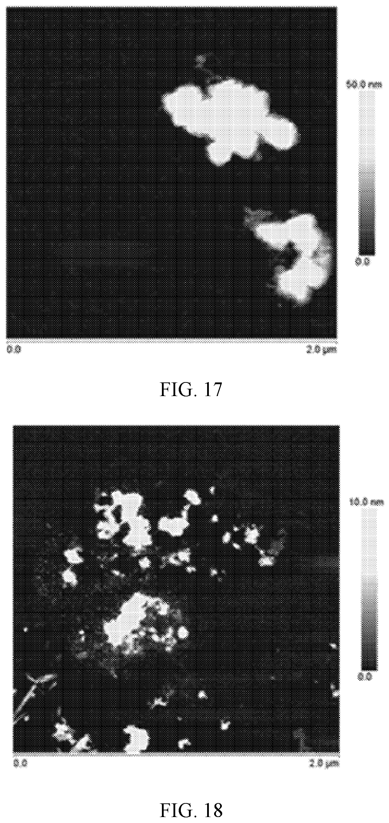

Using Aβ40 as a substrate, the substrate was mixed with Aβ42 in a certain proportion and aCSF was added to prepare a solution with an Aβ40 to Aβ42 ratio of 5 µM: 5 µM, the solution was incubated at 2000 rpm and 37°C, and used after incubation for 24 h. The aggregation morphology was determined by AFM to obtain Aβ aggregates with corresponding aggregation degree and morphology. FIG. 17 is an AFM morphology image of Aβ40 and Aβ42.

### Example 18

The method for preparing the Aβ oligomer in this embodiment is substantially the same as that in example 8, with partial adjustments made to the incubation conditions.

Using Aβ40 as a substrate, the substrate was mixed with Aβ42 in a certain proportion and aCSF was added to prepare a solution with an Aβ40 to Aβ42 ratio of 5 µM: 5 µM, the solution was incubated at 2000 rpm and 37°C, and used after incubation for 1 week. The aggregation morphology was determined by AFM to obtain Aβ aggregates with corresponding aggregation degree and morphology. FIG. 18 is an AFM morphology image of Aβ40 and Aβ42.

### Example 19

The method for preparing the Aβ oligomer in this example is performed with reference to the method for forming typical Aβ plaques reported in classical literature.

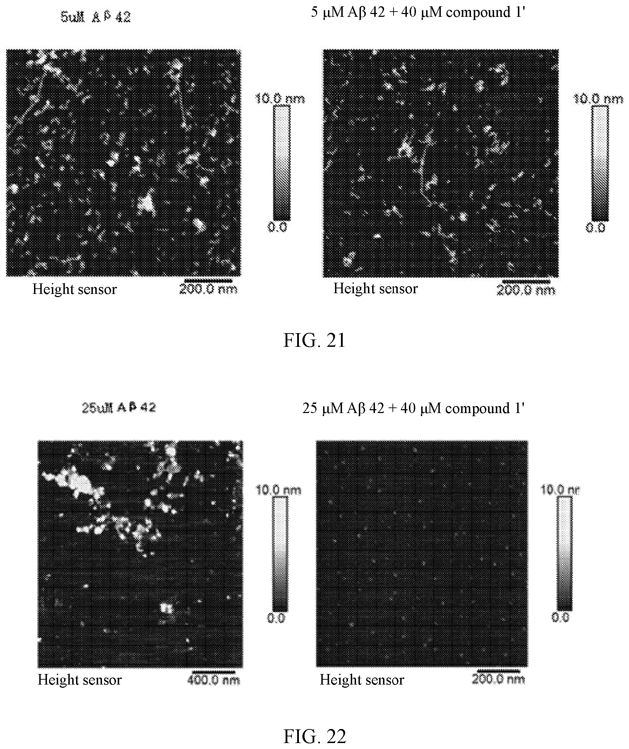

The DMSO stock solution with 5 mM Aβ prepared in (2) was taken, a mixed solution of 10 mM HCl + 150 mM NaCl was added to prepare a 100 µM Aβ solution, the solution was then diluted with the mixed solution of 10 mM HCl + 150 mM NaCl to 5 µM, incubated at 0 rpm and 37°C, and used after incubation for 48 h. The aggregation morphology was determined by AFM to obtain Aβ aggregates with corresponding aggregation degree and morphology. FIG. 21 is an AFM morphology image of Aβ42.

### Example 20

The method for preparing the Aβ oligomer in this embodiment is substantially the same as that in example 8, with partial adjustments made to the incubation conditions.

The DMSO stock solution with 5 mM Aβ prepared in (2) was taken, DPBS was added to prepare a 100 µM Aβ solution, the solution was then diluted with a DPBS buffer solution to 25 µM, incubated at 0 rpm and 37°C, and used after incubation for 78 h. The aggregation morphology was determined by AFM to obtain Aβ aggregates with corresponding aggregation degree and morphology. FIG. 22 is an AFM morphology image of Aβ42.

### Example 21

The method for preparing the Aβ oligomer in this embodiment is substantially the same as that in example 8, with partial adjustments made to the incubation conditions.

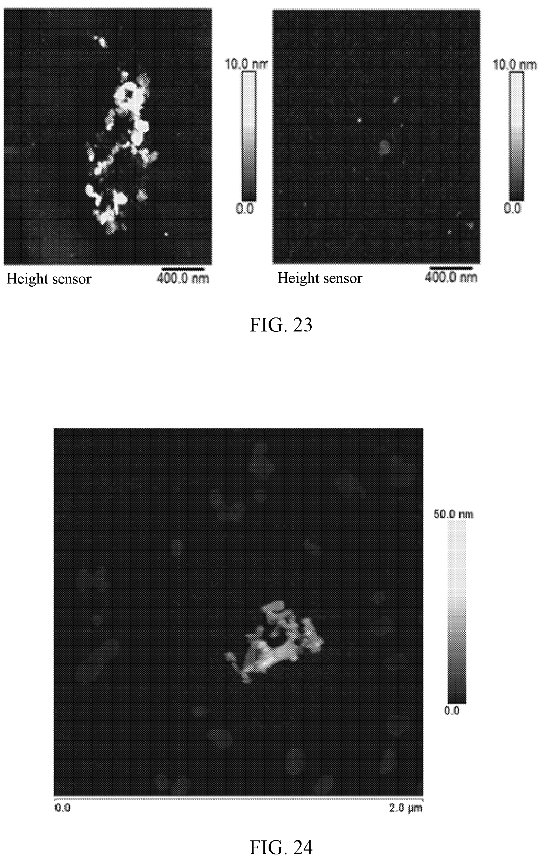

The DMSO stock solution with 5 mM Aβ38 prepared in (2) was taken, aCSF was added to prepare a 100 µM Aβ solution, the solution was then diluted with an aCSF buffer solution to 10 µM, incubated at 2000 rpm and 37°C, and used after incubation for 24 h. The aggregation morphology was determined by AFM to obtain Aβ aggregates with corresponding aggregation degree and morphology. FIG. 24 is an AFM morphology image of Aβ38.

### Example 22

The method for preparing the Aβ oligomer in this embodiment is substantially the same as that in example 8, with partial adjustments made to the incubation conditions.

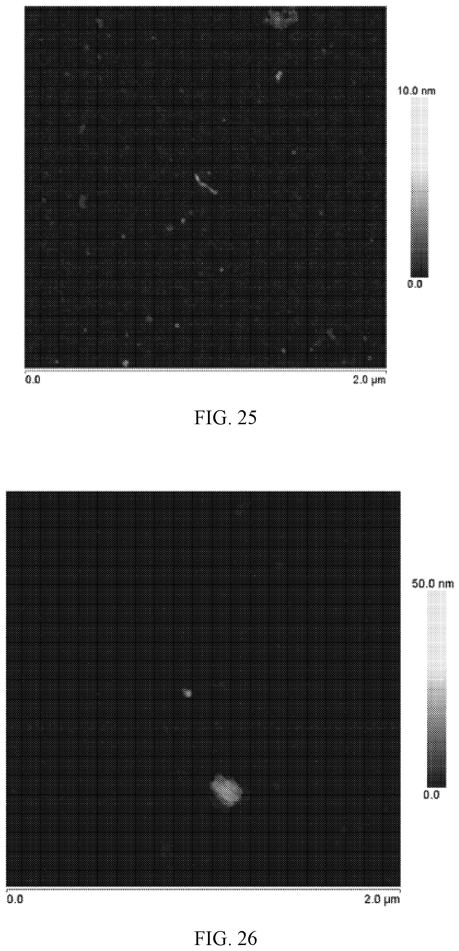

The DMSO stock solution with 5 mM Aβ38 prepared in (2) was taken, aCSF was added to prepare a 100 µM Aβ solution, the solution was then diluted with an aCSF buffer solution to 2 µM, incubated at 2000 rpm and 41°C, and used after incubation for 24 h. The aggregation morphology was determined by AFM to obtain Aβ aggregates with corresponding aggregation degree and morphology. FIG. 25 is an AFM morphology image of Aβ38.

### Example 23

The method for preparing the Aβ oligomer in this embodiment is substantially the same as that in example 8, with partial adjustments made to the incubation conditions.

The DMSO stock solution with 5 mM Aβ38 prepared in (2) was taken, Lysosome was added to prepare a 100 µM Aβ solution, the solution was then diluted with a Lysosome buffer solution to 2 µM, incubated at 2000 rpm and 41°C, and used after incubation for 24 h. The aggregation morphology was determined by AFM to obtain Aβ aggregates with corresponding aggregation degree and morphology. FIG. 26 is an AFM morphology image of Aβ38.

### Example 24

The method for preparing the Aβ oligomer in this embodiment is substantially the same as that in example 8, with partial adjustments made to the incubation conditions.

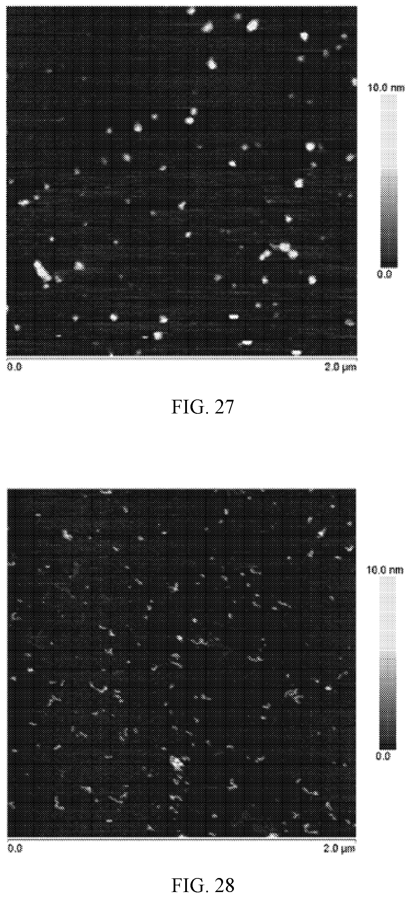

The Aβ DMSO stock solution with 5 mM Aβ42 prepared in (2) was taken, HBS was added to prepare a 100 µM Aβ solution, the solution was then diluted with an HBS buffer solution to 10 µM, incubated at 0 rpm and 37°C, and used after incubation for 24 h. The aggregation morphology was determined by AFM to obtain Aβ aggregates with corresponding aggregation degree and morphology. FIG. 27 is an AFM morphology image of Aβ42.

Compared with the incubated samples at a rotational speed of 2000 rpm under identical other conditions (right panel of FIG. 9), the samples incubated at 0 rpm exhibit a slightly lower degree of aggregation.

### Example 25

The method for preparing the Aβ oligomer in this embodiment is substantially the same as that in example 8, with partial adjustments made to the incubation conditions.

The DMSO stock solution with 5 mM Aβ40 prepared in (2) was taken, HBS was added to prepare a 100 µM Aβ solution, the solution was then diluted with an HBS buffer solution to 50 µM, incubated at 0 rpm and 37°C, and used after incubation for 24 h. The aggregation morphology was determined by AFM to obtain Aβ aggregates with corresponding aggregation degree and morphology. FIG. 28 is an AFM morphology image of Aβ40.

Compared with the incubated samples at a rotational speed of 2000 rpm under identical other conditions (left panel of FIG. 8), the samples incubated at 0 rpm exhibit a significantly lower degree of aggregation.

### Example 26

The method for preparing the Aβ oligomer in this embodiment is substantially the same as that in example 8, with partial adjustments made to the incubation conditions.

The DMSO stock solution with 5 mM Aβ40 prepared in (2) was taken, HBS was added to prepare a 100 µM Aβ solution, the solution was then diluted with an HBS buffer solution to 50 µM, incubated at 0 rpm and 37°C, and used after incubation for 1 week. The aggregation morphology was determined by AFM to obtain Aβ aggregates with corresponding aggregation degree and morphology. FIG. 28 is an AFM morphology image of Aβ40.

Compared with the incubated samples at a rotational speed of 2000 rpm under identical other conditions (left panel of FIG. 11), the samples incubated at 0 rpm exhibit a significantly lower degree of aggregation.

The following comparative example provides the method for preparing Aβ as disclosed in classic literature.

### Comparative Example 1

### Preparation of a typical Aβ monomer

An Aβ stock solution prepared in Example 8 was taken, ice water was added to a final Aβ concentration of 100 µM, and the mixture was vortexed for 15 s, and used immediately as an Aβ monomer.

### Comparative Example 2

### Preparation of a typical Aβ oligomer

A cold F12 culture medium was added to the Aβ stock solution to dilute Aβ to 100 µM, and the mixture was vortexed for 15 s, incubated at 4°C for 24 h to obtain the typical Aβ oligomer, as shown in the left panel of FIG. 7.

### Comparative Example 3

### Preparation of a typical Aβ fibril

10 mM HCl was added to the Aβ stock solution to dilute Aβ to 100 µM, and the mixture was vortexed for 15 s, incubated at 37°C for 24 h to obtain the typical Aβ fibril, where the typical Aβ fibril forms elongated and curved fibrous aggregates.

### Comparative Example 4

### Preparation of a typical Aβ plaque

10 mM HCl + 150 mM NaCl were added to the Aβ stock solution to enable the Aβ concentration to be 100 µM, and the mixture was vortexed for 15 s, incubated at 37°C for 24 h to obtain the Aβ plaque, as shown in the right panel of FIG. 7.

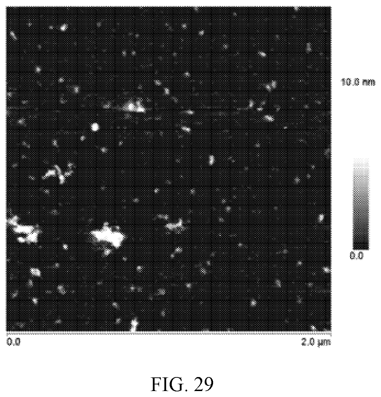

It can be known from FIG. 7, FIGS. 8-18, FIG. 21, FIG. 22, and FIGS. 24-29 that the morphology of the Aβ aggregates prepared by incubation under the extracellular matrix environment in human brain (physiological salt concentration and neutral condition) in examples 8 to 21 is different from that of typical Aβ plaques in the comparative examples. As shown in FIG. 8, FIG. 9, FIG. 11, FIG. 12, and FIG. 15, Aβ40 tends to form fibrous aggregates in its aggregated morphology, while Aβ42 is inclined to form small spherical-like aggregates and irregular lumps stacked therefrom. The morphologies of the Aβ40 and Aβ42 aggregates of the present invention are significantly different from those disclosed in classic literature of their respective comparative examples. The aggregation rates are also different. Only sporadic short fibrils can be observed for Aβ40 in literature even at a concentration of 100 µM, whereas fibrous aggregates can be formed within 24 h by Aβ40 at concentrations of 50 µM or even 10 µM under physiological salt concentration conditions (FIG. 8, FIG. 9 and FIG. 12). The fibril morphology is also distinct from that of Aβ40 and Aβ42 reported in the literature (The Journal of Biological Chemistry, 2002, 277(35): 32046-32053). Therefore, drug screening performed using these aggregates respectively will yield different screening results, which is similar to conducting drug screening against different targets. Screening using the Aβ aggregates prepared by simulating the in vivo microenvironment in the present invention possesses higher research value and application prospects.

### Test Example 1

The formation of the Aβ aggregates was detected via a ThT binding assay.

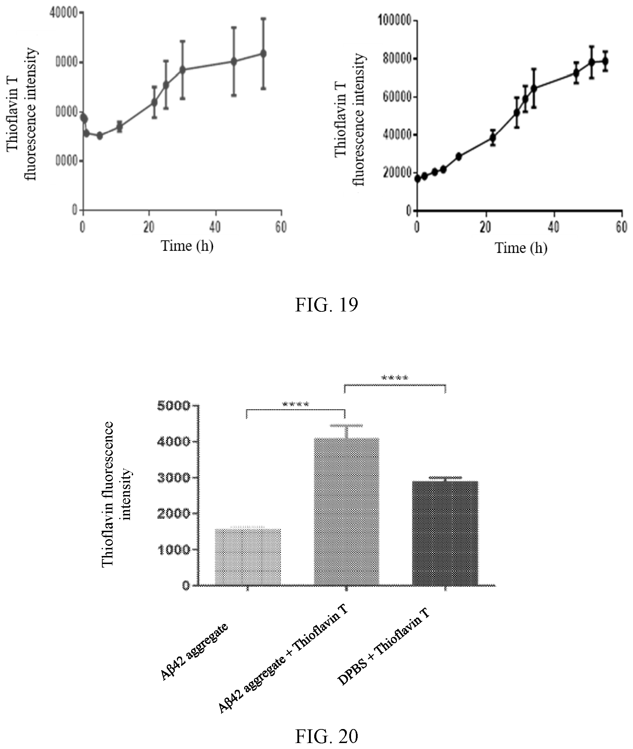

ThT can bind to the Aβ aggregates to produce enhanced fluorescence. The following experiments were conducted to monitor the aggregation degree of Aβ and the binding between ThT and Aβ aggregates respectively.
a) Kinetic monitoring of an aggregation process of Aβ: an initial Aβ solution (5-100 µM) formulated in the method in Example 21 was immediately added to a 96-well plate, a certain amount of ThT (5-100 µM) was added, and fluorescence values were measured at regular intervals using a fluorescence spectrophotometer (BioTek, Cytation 3) to monitor the aggregation process (Ex/Em: 440nm/490nm). Three parallel samples were arranged in each group. Statistical analysis was performed via GraphPad Prism, as shown in FIG. 19.
b) Fluorescence variation of addition of ThT to the Aβ aggregates: the Aβ aggregates prepared in Example 21 was added to the 96-well plate with the Aβ concentration ranging from 5 µM to 100 µM, fluorescence values were measured using a fluorescence spectrophotometer (BioTek, Cytation 3), the solution was incubated for 10-30 min after addition of ThT (a final concentration of 5-100 µM), and fluorescence values (Ex/Em:440 nm/490 nm) were measured again. Three parallel replicates were set for each group, and statistical analysis was performed via GraphPad Prism. As shown in FIG. 21, the fluorescence values were significantly increased after the ThT was added to the Aβ aggregates.

### Test Example 2

The morphology of each prepared Aβ aggregate was observed by an atomic force microscope (AFM).
a) Ultrapure water was injected into a syringe and filtered with a 0.02 mm filter, the initial 1-2 mL was discarded, and 0.02 mm filtered water was used in all subsequent steps.
b) Each Aβ sample was diluted to 10 µM or a sample with a final concentration less than 10 µM was directly used to prepare a sample to be measured on mica.
c) One to four layers of mica at the top was removed with an adhesive tape to expose the clean and flat surface of the mica.
d) A sample was added dropwise to the mica and allowed to stand for 30 s, rinsed with 200-400 µl of water, and blow-dried with compressed air.
e) The sample was placed in a clean cabinet at room temperature (The lid may be covered for dust prevention) until an analysis was performed.
f) Scanning was performed using a PeakForce tapping mode of Bruker FastScan atomic force microscope with a SNL-A probe. At least four regions were scanned to ensure representative sampling.
g) Data processing and image presentation were performed using NanoScope software.

### Test Example 3

Typical Aβ plaques and the Aβ aggregates prepared in the present invention were used to screen the active compound 1'.

According to the method for preparing the typical Aβ plaques in Comparative Example 4, under the formation conditions of the typical Aβ plaque (10 mM HCl + 150 mM NaCl, 37°C), even when the Aβ concentration was reduced from 100 µM to 5 µM, incubation with the compound 1' only exerted a slight inhibitory effect on Aβ aggregation, as shown in FIG. 21. This result indicates that the Aβ aggregates prepared under the solution conditions for forming the typical Aβ plaques cannot be used as targets to effectively screen out the Compound 1'.

In contrast, when incubated under conditions closer to the in vivo environment provided in Example 20 of the present invention (DPBS, 37°C, 0 rpm), the compound 1' at the same concentration can significantly inhibit Aβ aggregation even at a relatively high concentration of 25 µM. As shown in the right panel of FIG. 22, coincubation of the compound 1' with Aβ markedly suppresses the formation of Aβ aggregates. It is demonstrated that the Aβ aggregates of the present invention can serve as targets for screening the active compound 1 with more intuitive experimental results.

In this test example, the coincubation of the compound 1' and Aβ means that based on the method described in Comparative Example 4 or Example 20, the compound 1' is added into the Aβ stock solution and mixed with Aβ stock solution for incubation, so that the concentration of the compound 1' reaches the concentration required for the experiment.

Furthermore, the inventors further found that after the Aβ aggregates prepared in the examples were coincubated with the compound for a certain period of time, the aggregation states before and after compound addition were compared via AFM observation. The compound 1 can also significantly promote the disaggregation of Aβ aggregates, and representative experimental results are shown in FIG. 23.

### Example 27 Molecular biological assessment on the therapeutic efficacy of the compound 1' in AD model mice

To evaluate the therapeutic efficacy of the compound 1' in the AD model mice, we have performed systematic molecular biological experiments. Specifically, Aβ immunofluorescence staining and microglial immunostaining were employed to assess pathological alterations in the brains of AD model mice following four months of treatment. The evaluation includes detailed analysis of changes in the number of Aβ plaques per unit area and the area of activated microglia in the brain.

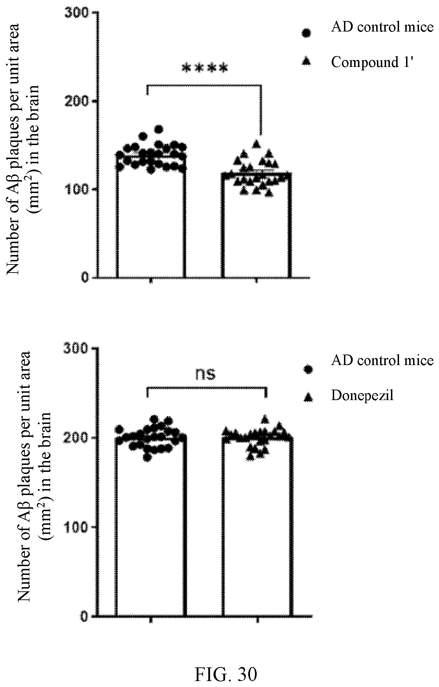

Aβ immunofluorescence staining assay: sections were prepared from brain tissues. Mice were anesthetized with Avertin and perfused with PBS, and brain tissues were taken out and placed in 4% PFA overnight. After dehydration in 15% and 30% sucrose solutions, the brain tissues were embedded, and 30 µm thick sections were cut using a cryostat. The sections were placed in a blocking solution (a PBS solution containing 10% of bovine serum albumin and 0.3% of Triton X-100) and incubated at 37 °C for 30 min. Subsequently, the sample was incubated with a primary antibody overnight at 4°C. The primary antibody against Aβ (6E10) was diluted at 1: 500 in the PBS solution containing 3% of bovine serum albumin and 0.3% of Triton X-100. After removal at 4°C, the sections were allowed to warm to room temperature for at least 10 min. The primary antibody was removed, and the sections were washed three times with PBS (10 min per wash). Next, the sections were incubated with a mouse-647 secondary antibody in the dark 37°C for 1 h, where a dilution solution was PBS and a dilution ratio was 1: 1000. Images were acquired using a fluorescence microscope to quantitatively evaluate the number of Aβ plaques per unit area in the mouse brain. Experimental results showed that after 4 months of treatment with the compound 1' (intraperitoneal injection once-daily at a dose of 10 mg/kg, dissolved in a 0.5% CMCNa aqueous solution), the area of Aβ plaques in the brain of Alzheimer's disease (AD) model mice was significantly reduced (as shown in FIG. 30 and Tables 2 and 3). In comparison, after 7 months of treatment with Donepezil (at a daily oral dose of 2 mg/kg), no significant changes were observed in the number of Aβ plaques in mice of the positive control group (as shown in FIG. 30 and the table below). These results strongly indicated that the compound 1' could effectively reduce formation of Aβ plaques within a shorter time, thereby demonstrating its potential advantages in the treatment of AD.

**Table 2: the number of Aβ plaques per unit area in the brain after 4 months of administration of the compound 1' to 6-month-old APP/PS1 mice**

| Group | Number of Aβ plaques per unit area (mm²) in the brain | |
|---|---|---|
| | Mean value | Variance |
| AD control | 138.9 | 11.9 |
| Compound 1' treatment group | 118.8 | 14.3 |

**Table 3: The number of Aβ plaques per unit area in the brain after 7 months of administration of Donepezil to 6-month-old APP/PS1 mice**

| Group | Number of Aβ plaques per unit area (mm²) in the brain | |
|---|---|---|
| | Mean value | Variance |
| AD control | 200.4 | 10.6 |
| Donepezil treatment group | 200.6 | 9.6 |

Immunostaining assay of microglia: Mice were anesthetized with Avertin and perfused with PBS, and brain tissues were taken out and placed in 4% PFA overnight. After dehydration in 15% and 30% sucrose solutions, the brain tissues were embedded, and 30 µm thick sections were cut using a cryostat. The sections were placed in a blocking solution (a PBS solution containing 10% of bovine serum albumin and 0.3% of Triton X-100) and incubated at 37 °C for 30 min. Subsequently, the sample was incubated with a primary antibody overnight at 4°C. The primary antibody (Iba1) activated microglia was diluted at 1: 500 in the PBS solution containing 3% of bovine serum albumin and 0.3% of Triton X-100. After removal at 4°C, the sections were allowed to warm to room temperature for at least 10 min. The primary antibody was removed, and the sections were washed three times with PBS (10 min per wash). Next, the sections were incubated with a rabbit-488 secondary antibody in the dark at 37°C for 1 h, where a dilution solution was PBS and a dilution ratio was 1: 1000.

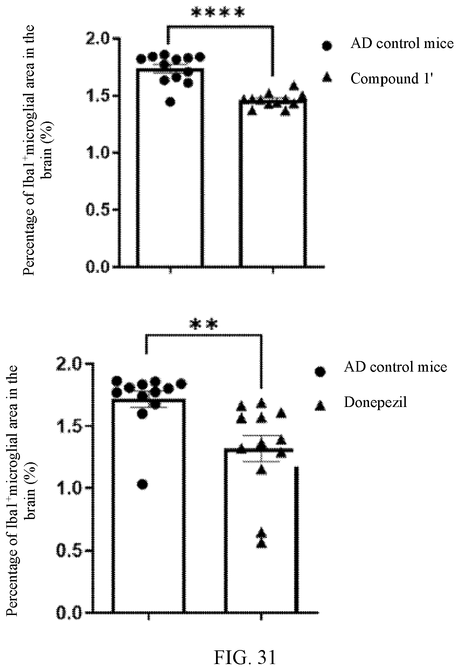

Fluorescence microscopic observation and analysis were performed to evaluate the quantity and distribution of activated microglia. Experimental results showed that after 4 months of treatment with the compound 1' (intraperitoneal injection once-daily at a dose of 10 mg/kg, dissolved in a 0.5% CMCNa aqueous solution), the area of the activated microglia in the brain of Alzheimer's disease (AD) mice was significantly reduced (as shown in FIG. 31 and Table 4). Similarly, in the group treated with the positive drug donepezil (at a daily oral dose of 2 mg/kg), a significant reduction in the area proportion of the activated microglia in the brain was also observed (as shown in FIG. 31 and Table 5). This result showed that both the compound 1' and donepezil exerted significant effects on reducing the activation level of microglia in the brain of AD model mice.

**Table 4: the area proportion of the activated microglia in the brain after 4 months of administration of the compound 1' to 6-month-old APP/PS1 mice**

| Group | Percentage of Iba1+ microglial area in the brain | |
|---|---|---|
| | Mean value | Variance |
| AD control | 1.74 | 0.13 |
| Compound 1' treatment group | 1.46 | 0.06 |

**Table 5: the area proportion of the activated microglia in the brain after 7 months of administration of donepezil to 6-month-old APP/PS1 mice**

| Group | Percentage of the Iba1+ microglial area in the brain | |
|---|---|---|
| | Mean value | Variance |
| AD control | 1.71 | 0.25 |
| Donepezil treatment group | 1.35 | 0.32 |

### Example 28 Behavioral evaluation of therapeutic effects of the compound 1' on the AD model mice

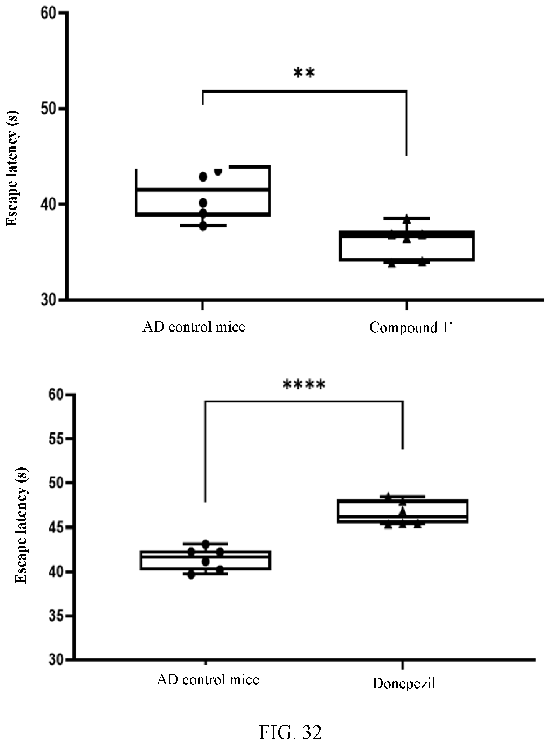

The present invention focused on evaluating the cognitive function improvement effect of the compound 1' in the Alzheimer's disease (AD) model mice, specifically 6-month-old APP/PS1 mice. Systematic administration of the compound was carried out for up to 7 months (once-daily intraperitoneal injection at a dose of 10 mg/kg, dissolved in a 0.5% CMCNa aqueous solution). At the end of the treatment period, by comparing the performances in the Morris water maze test between mice in the compound 1' treatment group and the control group administered with an equal volume of 0.5% CMCNa aqueous solution, the speed and path length of mice reaching the platform were recorded in detail to analyze the intervention effect of the compound on spatial learning and memory abilities. Results showed that after 7-month treatment, the AD model mice exhibited significant improvements in spatial learning and memory functions (as shown in FIG. 32 and Tables 6 and 7). It was worth noting that in the control group treated with donepezil (at a daily oral dose of 2 mg/kg, dissolved in water), the long-term memory ability was decreased compared with that of the solvent control group. This result highlighted the potential superiority of the compound 1' in ameliorating AD-associated cognitive impairment.

**Table 6: Morris water maze test results after 7 months of administration of the compound 1' to 6-month-old APP/PS1 mice**

| Group | Escape latency (S) | |
|---|---|---|
| | Mean value | Variance |
| AD control | 41.5 | 3.0 |
| Compound 1' treatment group | 36.1 | 1.79 |

**Table 7: Morris water maze test results after 7 months of administration of donepezil to 6-month-old APP/PS1 mice**

| Group | Escape latency (S) | |
|---|---|---|
| | Mean value | Variance |
| AD control | 41.4 | 1.3 |
| Donepezil treatment group | 46.6 | 1.38 |

### Example 29 PET imaging evaluation of therapeutic effects of the compound 1' in the AD model mice

PET imaging technology exhibits unique advantages in detecting the therapeutic effect of the compound 1' on the AD models. As a highly sensitive molecular imaging tool, PET enables real-time and non-invasive detection of in vivo pathological changes, providing accurate quantitative data for in vivo research. By using specific radioactive probes such as [¹⁸F]AV45 and [¹⁸F]DPA714, PET imaging can respectively quantify Aβ plaque deposition and neuroinflammation levels induced by microglial TSPO expression, which are closely associated with the core pathological characteristics of AD. Compared with traditional tissue sectioning or in vitro analytical methods, PET imaging can dynamically assess changes in the same individual animal before and after treatment, reduce animal usage and improve the reliability of experimental results. Therefore, this study adopts the combined application of Aβ probe [¹⁸F]AV45 and TSPO probe [¹⁸F]DPA714 to evaluate alterations in pathological features and inflammatory responses, thereby realizing dynamic tracking of pathological status in living animals.

Animal models: APP/PS1 double transgenic mice (AD model) and age- and gender-matched normal C57BL/6J control mice were selected. The APP/PS1 mice were randomly divided into the treatment group (treated with compound 1') and the model group (administered with normal saline). Each group of mice was injected with approximately 10 MBq of [¹⁸F]AV45 or [¹⁸F]DPA714 for PET imaging.

PET imaging method: by injecting [¹⁸F]AV45 (for detecting Aβ deposition) or [¹⁸F]DPA714 (for detecting microglial TSPO expression) via the tail vein, a 15-minute static PET scan was performed 45 min after injection, with mice anesthetized with 1-2% isoflurane during scanning.

Data processing: PET data was reconstructed by using a 3D OSEM method, and attenuation, scattering, normalization and detector dead time correction were performed. PET images were aligned with mouse brain templates using Pmod software for precise localization and segmentation of brain regions. Reference brain regions and data analysis: the [¹⁸F] AV45 probe selected the cerebellum as a reference brain region, the [¹⁸F] DPA714 probe selected the hypothalamus as a reference brain region, SUVR values in different brain regions were calculated, and the effects of the compound 1' on Aβ deposition and neuroinflammation were analyzed.

### Therapeutic efficacy evaluation:

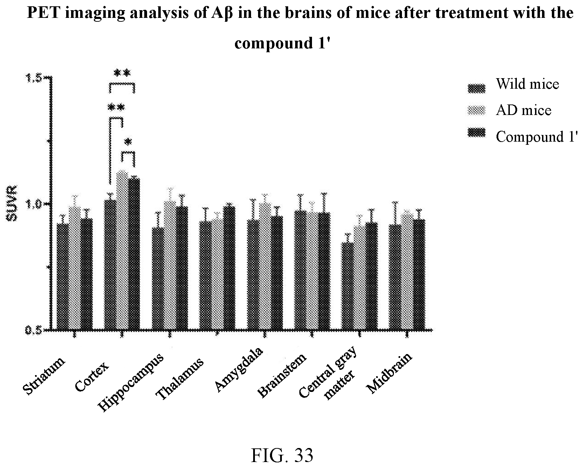

Changes in Aβ deposition: The uptake ratio of [¹⁸F]AV45 in the hippocampus and cortex of mice in the treatment group was significantly lower than that in the model group, indicating that the compound 1' effectively reduced Aβ plaque deposition (as shown in FIG. 33 and Table 8).

**Table 8**

| Organ | SUVR | | | | | | | | | | |
|---|---|---|---|---|---|---|---|---|---|---|---|
| | Wild | | | | AD model mice | | | Treatment group | | | |
| Striatum | 0.90 | 0.88 | 0.96 | 0.94 | 0.94 | 1.02 | 1.01 | 0.94 | 0.93 | 0.99 | 0.91 |
| Cortex | 1.01 | 1.03 | 1.03 | 0.98 | 1.12 | 1.12 | 1.13 | 1.09 | 1.11 | 1.10 | 1.10 |
| Hippocampus | 0.86 | 0.86 | 0.98 | 0.93 | 1.07 | 0.97 | 0.99 | 1.01 | 0.94 | 1.04 | 0.97 |
| Thalamus | 0.92 | 0.87 | 1.00 | 0.94 | 0.91 | 0.95 | 0.96 | 1.00 | 0.99 | 1.00 | 0.98 |
| Amygdala | 0.91 | 0.84 | 1.00 | 1.00 | 0.99 | 0.98 | 1.04 | 0.91 | 0.99 | 0.97 | 0.94 |
| Brainstem | 0.95 | 0.90 | 1.03 | 1.02 | 0.98 | 0.93 | 1.00 | 0.86 | 1.00 | 0.98 | 1.03 |
| Central gray matter | 0.88 | 0.82 | 0.82 | 0.87 | 0.87 | 0.91 | 0.96 | 1.00 | 0.92 | 0.92 | 0.87 |
| Midbrain | 0.90 | 0.81 | 0.99 | 0.98 | 0.95 | 0.97 | 0.96 | 0.90 | 0.92 | 0.96 | 0.98 |

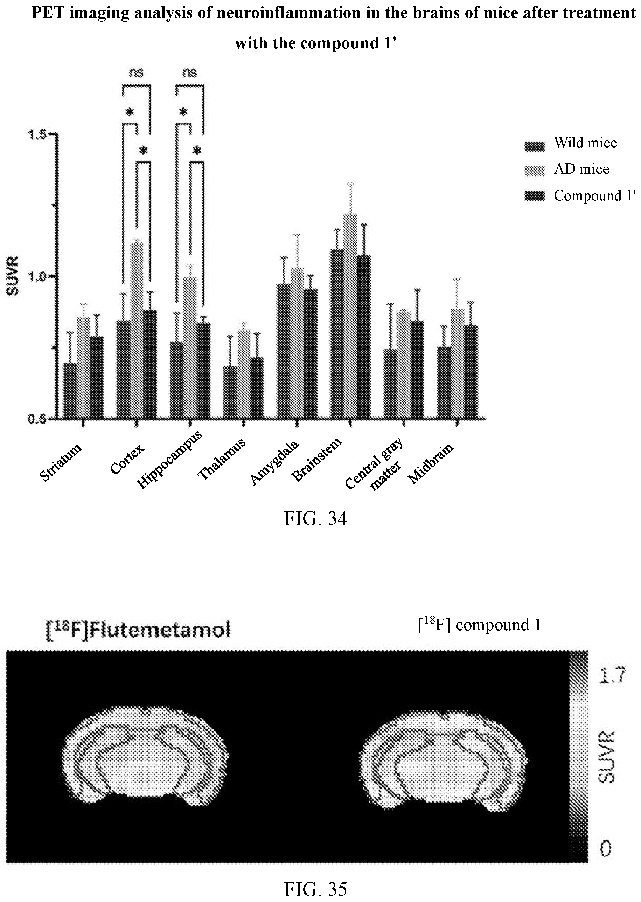

Neuroinflammation level: The uptake of [¹⁸F]DPA714 in the hippocampus and cortex of mice in the treatment group was also significantly lower than that in the model group, indicating that the compound 1' could inhibit microglial activation and alleviate neuroinflammation (as shown in FIG. 34 and Table 9).

**Table 9**

| Organ | SUVR | | | | | | | | | |
|---|---|---|---|---|---|---|---|---|---|---|
| | Wild | | | | AD model mice | | | Treatment group | | |
| Striatum | 0.58 | 0.69 | 0.68 | 0.84 | 0.817 | 0.905 | 0.844 | 0.79 | 0.86 | 0.71 |
| Cortex | 0.76 | 0.88 | 0.78 | 0.96 | 1.126 | 1.126 | 1.100 | 0.84 | 0.85 | 0.95 |
| Hippocampus | 0.64 | 0.80 | 0.76 | 0.88 | 0.973 | 1.046 | 0.963 | 0.84 | 0.86 | 0.81 |
| Thalamus | 0.53 | 0.70 | 0.72 | 0.78 | 0.824 | 0.824 | 0.789 | 0.74 | 0.78 | 0.62 |
| Amygdala | 1.04 | 0.84 | 1.04 | 0.97 | 0.974 | 1.164 | 0.956 | 0.96 | 1.00 | 0.91 |
| Brainstem | 1.17 | 1.01 | 1.12 | 1.08 | 1.281 | 1.281 | 1.099 | 1.16 | 1.11 | 0.95 |
| Central gray matter | 0.54 | 0.74 | 0.77 | 0.93 | 0.881 | 0.881 | 0.873 | 0.88 | 0.93 | 0.72 |
| Midbrain | 0.65 | 0.76 | 0.80 | 0.80 | 0.818 | 1.006 | 0.836 | 0.86 | 0.89 | 0.74 |

### Example 30: (Comparison of PET imaging between [¹⁸F]-compound 1 and control drug [¹⁸F]-Flutemetamol)

About 10 MBq of [¹⁸F] compound 1 and control drug [¹⁸F] Flutemetamol were intravenously injected to the APP/PS1 mice and wild-type mice (with same age and gender). Static PET scanning was performed 45 min after injection, with a scanning duration of 15 min. During scanning, the mice were anesthetized with 1-2% isoflurane. PET data was reconstructed by using a 3D OSEM method, and attenuation, scattering, normalization and detector dead time correction were performed. The corrected PET images were co-registered with CT images. The reconstructed data was aligned with the standardized mouse brain template via Pmod 4.3 software to achieve precise localization and segmentation of brain regions. The cerebellum was selected as the reference region to compare the standardized uptake value ratio (SUVR) of [¹⁸F] compound 1 and the control drug [¹⁸F]Flutemetamol in the brain tissues of APP/PS1 mice and wild-type mice. Data results were shown in FIG. 35 and Table 10. The SUVR of [¹⁸F] compound 1 in cerebral cortex and hippocampal tissues was significantly higher than that of the control drug [¹⁸F]Flutemetamol, indicating that [¹⁸F] compound 1 exhibited superior imaging efficacy compared with [¹⁸F]Flutemetamol.

**Table 10**

| | SUVR (reference tissue cerebellum) | | |
|---|---|---|---|
| | | Cortex | Hippocampus |
| APP/PS1 mice | [¹⁸F] compound 1 | 1.21 | 1.07 |
| | [¹⁸F]Flutemetamol | 1.12 | 0.97 |
| Wild mice | [¹⁸F] compound 1 | 0.98 | 0.86 |
| | [¹⁸F]Flutemetamol | 1.03 | 0.85 |

## Claims

1. A compound represented by formula D, a pharmaceutically acceptable salt thereof, a solvate thereof, or a solvate of the pharmaceutically acceptable salt thereof: 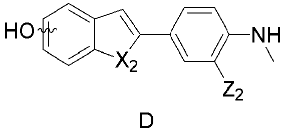 wherein X₂ is O, S, or Se;
Z₂ is F-18 or I-124.

2. The compound, the pharmaceutically acceptable salt thereof, the solvate thereof, or the solvate of the pharmaceutically acceptable salt thereof according to claim 1, wherein, satisfying one or more of the following conditions:
(1) X₂ is O or S;
(2) Z₂ is F-18;
(3) the compound represented by formula D is 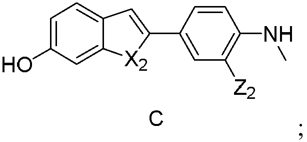 preferably, the compound represented by formula D is

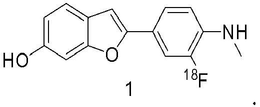

3. A compound represented by formula E, a pharmaceutically acceptable salt thereof, a solvate thereof, or a solvate of the pharmaceutically acceptable salt thereof, wherein: 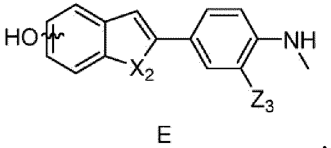 wherein X₂ is as defined in claim 1 or 2;
Z₃ is F or I, for example F;
preferably, the compound represented by formula E is

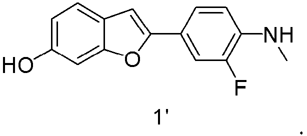

4. A compound represented by formula A, a pharmaceutically acceptable salt thereof, a solvate thereof, or a solvate of the pharmaceutically acceptable salt thereof: 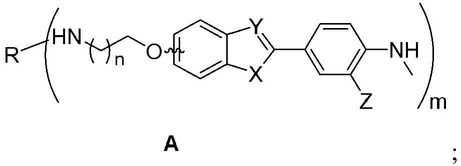
wherein R is composed of a chelating group and a radionuclide;
the O atom is linked to a linkage position on a benzene ring or a five-membered ring;
the chelating group is unmodified or modified with a cell-penetrating peptide;
the radionuclide is a diagnostic radionuclide;
m is 1 or 2;
each X is independently O, S, or Se;
each Y is independently CH or N;
each Z is independently H, F, F-18, Cl, Br, or I;
each n is independently 1, 2, 3, or 4.

5. The compound, the pharmaceutically acceptable salt thereof, the solvate thereof, or the solvate of the pharmaceutically acceptable salt thereof according to claim 4, satisfying one or more of the following conditions:
(1) X is O or S;
(2) Z is F or H;
(3) n is 3 or 4;
(4) the cell-penetrating peptide is HAIYPRH, THRPPMWSPVWP, TFFYGGSRGKRNNFKTEEY, AGILKRW, CGNKRTR, LRKLRKRLLR, or YGRKKRRQRRR;
(5) the chelating group is 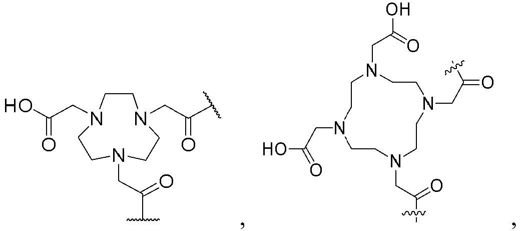 or 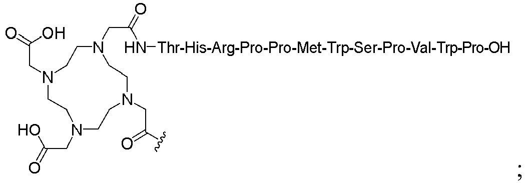 and
(6) the radionuclide is selected from Cu-64 and Ga-68, for example Ga-68.

6. The compound, the pharmaceutically acceptable salt thereof, the solvate thereof, or the solvate of the pharmaceutically acceptable salt thereof according to claim 4, wherein the compound represented by formula A is represented by formula A-1, wherein R, X, Y, Z, and n are as defined in any one of claims 4 to 5;

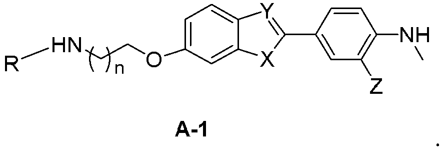

7. The compound, the pharmaceutically acceptable salt thereof, the solvate thereof, or the solvate of the pharmaceutically acceptable salt thereof according to claim 4, wherein the compound represented by formula A is selected from any one of the following compounds:

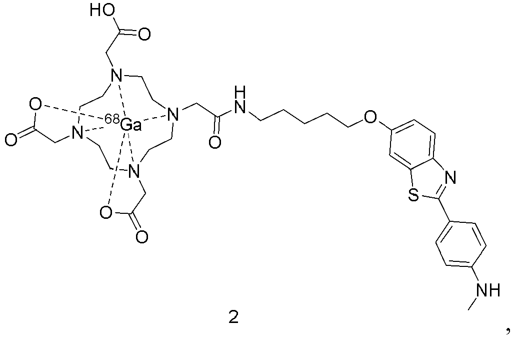

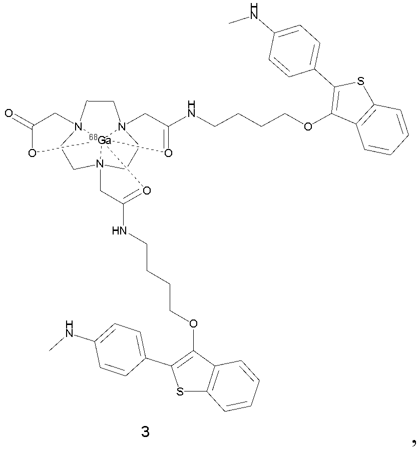

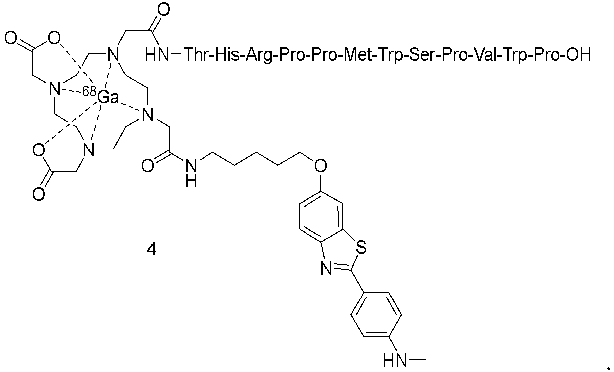

8. A compound represented by formula B, a pharmaceutically acceptable salt thereof, a solvate thereof, or a solvate of the pharmaceutically acceptable salt thereof: 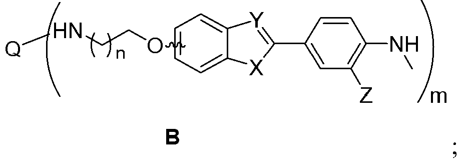 wherein X, Y, Z, m, and n are as defined in any one of claims 4 to 7, and Q is a chelating group as defined in any one of claims 4 to 7.

9. The compound, the pharmaceutically acceptable salt thereof, the solvate thereof, or the solvate of the pharmaceutically acceptable salt thereof according to claim 8, wherein the compound represented by formula B is any one of the following compounds:

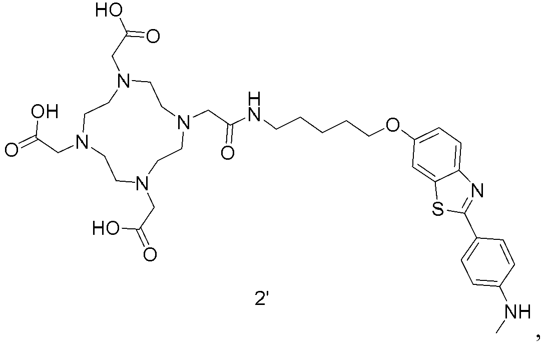

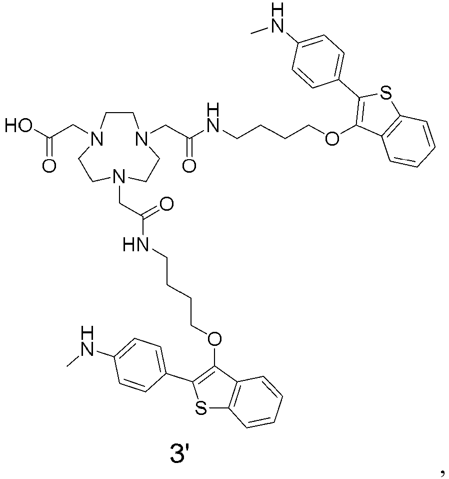

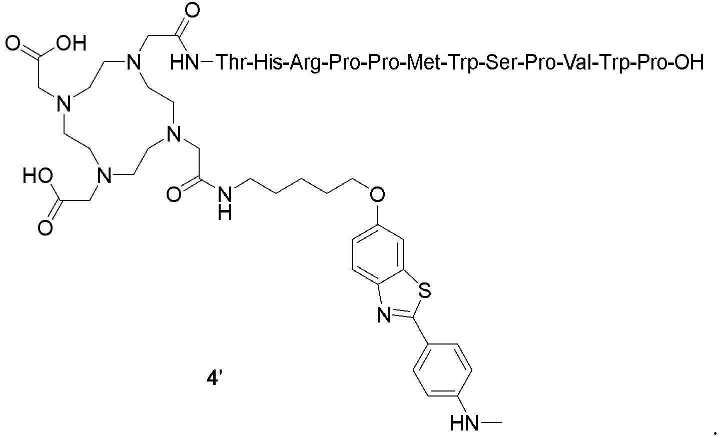

10. A compound represented by formula C, a pharmaceutically acceptable salt thereof, a solvate thereof, or a solvate of the pharmaceutically acceptable salt thereof: 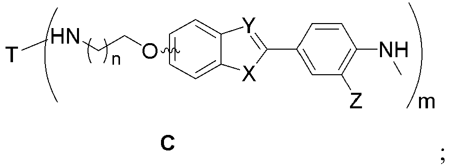
wherein X, Y, Z, m, and n are as defined in any one of claims 4 to 7;
T is composed of a chelating group and a non-radionuclide, and the chelating group is as defined in any one of claims 4 to 7.

11. The compound, the pharmaceutically acceptable salt thereof, the solvate thereof, or the solvate of the pharmaceutically acceptable salt thereof according to claim 10, wherein the non-radionuclide is Cu or Ga, for example Ga;
preferably, the compound represented by formula C is

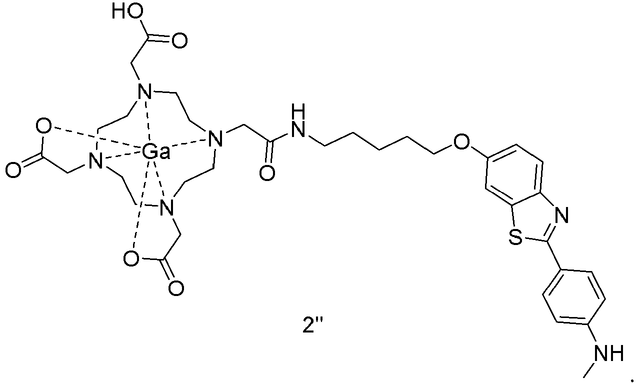

12. A pharmaceutical composition, comprising the compound, the pharmaceutically acceptable salt thereof, the solvate thereof, or the solvate of the pharmaceutically acceptable salt thereof according to any one of claims 1 to 11, and a pharmaceutically acceptable excipient.

13. A kit, comprising a substance V and instructions for use, wherein the substance V is the compound, the pharmaceutically acceptable salt thereof, the solvate thereof, or the solvate of the pharmaceutically acceptable salt thereof according to any one of claims 1 to 11, or the pharmaceutical composition according to claim 12.

14. Use of a substance W in preparation of a PET molecular probe, wherein the substance W is the compound represented by formula A, the pharmaceutically acceptable salt thereof, the solvate thereof, or the solvate of the pharmaceutically acceptable salt thereof according to any one of claims 4 to 7, or the compound represented by formula D, the pharmaceutically acceptable salt thereof, the solvate thereof, or the solvate of the pharmaceutically acceptable salt thereof according to claim 1 or 2;
preferably, the PET molecular probe is used for diagnosis of Alzheimer's disease; more preferably, the PET molecular probe is used for imaging Aβ protein-binding.

15. Use of a substance V in preparation of a medicament for preventing and/or treating Alzheimer's disease, wherein the substance V is the compound, the pharmaceutically acceptable salt thereof, the solvate thereof, or the solvate of the pharmaceutically acceptable salt thereof according to any one of claims 1 to 11, or the pharmaceutical composition according to claim 12; preferably, the substance V is the compound represented by formula D, the pharmaceutically acceptable salt thereof, the solvate thereof, or the solvate of the pharmaceutically acceptable salt thereof according to claim 1 or 2, or a pharmaceutical composition comprising the compound represented by formula D, the pharmaceutically acceptable salt thereof, the solvate thereof, or the solvate of the pharmaceutically acceptable salt thereof according to claim 1 or 2, and a pharmaceutically acceptable excipient.

16. Use of a substance V in preparation of an anti-Aβ drug, wherein the substance V is as defined in claim 15; preferably, the anti-Aβ drug exerts a therapeutic effect by disaggregating Aβ oligomers or by preventing the formation of Aβ oligomers.

17. A substance V performing one or more of the following functions: (i) inhibiting formation of Aβ oligomers; (ii) promoting disaggregation of Aβ oligomers; (iii) binding to Aβ oligomers, wherein the substance V is as defined in claim 15.

18. A method for preparing an Aβ aggregate, the method comprising the following steps:
S1, dissolving an Aβ lyophilized powder and drying to remove a solvent to obtain an Aβ peptide film;
S2, mixing the Aβ peptide film with a buffer solution to obtain an Aβ solution with a concentration of 0.2-100 µM;
S3, incubating the Aβ solution to obtain an Aβ aggregate; and
wherein in step S1, the Aβ lyophilized powder is Aβ40, Aβ42, N3pE42, Aβ38, or a combination thereof;
in step S2, the buffer solution is selected from at least one of calcium- and magnesium-free Dulbecco's phosphate-buffered saline, an HBS buffer solution containing 8-12 mM 4-(2-hydroxyethyl)-1-piperazineethanesulfonic acid (HEPES) and 140-160 mM NaCl, artificial cerebrospinal fluid, DMEM, FBS, endosome-mimicking fluid, and lysosome-mimicking fluid;
in step S3, the incubation temperature is 30 to 43°C, the incubation time is not less than 1 day, and the incubation shaking speed is 0 to 2500 rpm.

19. The method according to claim 18, wherein in step S3, the incubation time is 1 to 7 days.

20. The method according to claim 18, wherein in step S3, the incubation is performed in a constant-temperature homogenizer, and a rotational speed of the constant-temperature homogenizer is 0 to 2500 rpm.

21. The method according to claim 18, wherein the buffer solution is the calcium- and magnesium-free Dulbecco's phosphate-buffered saline (DPBS), the HBS buffer solution containing 8-12 mM HEPES and 140-160 mM NaCl, or the artificial cerebrospinal fluid (aCSF).

22. A target for screening an anti-Aβ drug, wherein the target is an Aβ aggregate, and the Aβ aggregate is prepared by the method according to any one of claims 18 to 21.

23. Use of an Aβ aggregate as a target for screening an anti-Aβ drug, wherein the Aβ aggregate is prepared by the method according to any one of claims 18 to 21.

24. A method for screening an anti-Aβ drug, comprising the following step: screening the anti-Aβ drug using the target according to claim 22.

25. The method according to claim 24, wherein a drug capable of disaggregating Aβ aggregates or inhibiting Aβ aggregation to form aggregates is screened, the drugis selected from at least one of an antibody, a polypeptide, and a small molecule compound.

26. An anti-Aβ drug obtained by the method according to claim 24 or 25.

27. Use of a compound represented by formula I, for in vitro non-therapeutic use in (i) inhibiting formation of Aβ oligomers; (ii) promoting disaggregation of Aβ oligomers; and/or (iii) binding to Aβ oligomers;
